# EUROPEAN PATENT APPLICATION

(11) **EP 1 243 646 A2**
(43) Date of publication of application: **25.09.2002**
(21) Application number: 02290571.5
(22) Date of filing: 07.03.2002
(51) Int. Cl.: C12N 15/00, A01K 67/033, C07K 14/47, A61K 49/00

(54) **Transgenic Drosophila with human APC gene for pharmacological screenings**

(30) Priority: 19.03.2001 US 276483 P
(71) Applicant: Council of Scientific and Industrial Research, New Delhi 110 001 (IN)
(72) Inventor: Bhandari, Poonam, Hyderabad 500 007 (IN); Shashidhara, L.S., Hyderabad 500 007 (IN)
(74) Representative: Bloch, Gérard

(57) **Abstract**

The present invention relates to the development of an *in vivo* assay system, useful for developing therapeutic options by testing the efficacy of drugs and other substances, said system constituting a novel, fast, and inexpensive *in vivo* assay system wherein, transgenic *Drosophila* harbors human Adenomatous Polyposis Coli gene, a negative regulator of β-catenin.

## Description

### Field of the present invention

The present invention relates to the development of an *in vivo* assay system. The said system is useful for developing therapeutic options. The drugs and other substances are tested for their efficacy using said system. The said system constitutes a novel, fast, and inexpensive *in vivo* assay system. The said system is consisting of transgenic *Drosophila* harboring human Adenomatous Polyposis Coli gene, a negative regulator of β-catenin.

### Background and prior art references of the invention

Studies over the decades have identified a number of genes that are associated with the transformation of normal cells to cancer cells and the progression of benign tumors to malignant tumors.

For prevention, one would like to target drugs to genes that are altered early in the process, whereas gene that are altered at various stages of cancer progression form targets for therapeutic drug selection.¹

Identification of suitable targets for drug screening and validation of a potential drug as specific modifier of a given target requires simple model systems amenable to various kinds of genetic and *in vivo* biochemical analyses.

β-Catenin, a transducer of Wnt signaling, is a potential oncogene, activation of which is the cause of many different kinds of cancer, such as colon cancer, melanomas, ovarian cancer, medulloblastoma, prostate cancer, uterine cancer etc. In the absence of Wnt signaling, free cytoplasmic β-catenin is subjected to ubiquitin-mediated protein degradation.

According to the current model of β-catenin regulation, APC (protein product of human Adenomatous Polyposis Coli gene) forms a complex with β-catenin, which is then recruited, by Axin/GSK-3β complex. This complex formation allows β-catenin to be phosphorylated by GSK-3β and the phosphorylated β-catenin is the target of ubiquitin-mediated degradation pathway.

Stabilization of β-catenin is caused by gain-of-function mutations in *wnt-1* proto-oncogene or loss-of-function mutations in tumor-suppressor genes *APC* (US patent - 5,736,389) or *Axin*. Direct oncogenic activation of β-catenin is also reported, which is caused by specific mutations in β-catenin that prevent its phosphorylation by GSK-3β.

Method of producing transgenic Typhoid fly is discussed for MUSCA DOMESTICA (Russian Patent No.- 2137362). Also, APC function, especially the down regulation of β-catenin appears to be conserved between *Drosophila* and humans (Hayashi et al., 1997; Ahmed et al., 1998).

In addition, *Drosophila* homologue of APC, dAPC can mediate degradation of β-catenin in colon epithelial cells (Hayashi et al., 1997).

Study is been done on an in situ insect brain tissue assay, particularly suited for Dipteran brain tissue. The assay centres on the observation by the present inventors that calcium levels in the brain of Drosophila appear to oscillate when detected using a system employing transgenic apoaequorin to measure intracellular levels of calcium in the brain tissue.

Extensive biochemical analysis of APC protein, mostly *in vitro* and cell culture studies, has resulted in the identification of binding sites for a number of proteins. Most important of them, in the context of tumorigenesis, is the central region that harbors β-catenin binding domains (Polakis, 1997).

Cell lines with mutations in the *APC* gene show enhanced levels of β-catenin, which suggest that APC negatively regulates the cytoplasmic levels of β-catenin, increase in which may affect Wnt/Wg signaling and/or cell adhesion. It has been shown that in the absence of the Wnt/Wg signaling, the cytoplasmic β-catenin is phosphorylated and targeted for ubiquitin-proteosome mediated degradation (Polakis, 1997; Polakis, 1999). As β-catenin does not directly interact with GSK-3 β, the formation of a complex consisting of GSK-3 β, Axin and APC facilitates its phosphorylation and subsequent degradation.

Recently, it has been hypothesized that APC is involved in the transportation of unphosphorylated β-catenin to the degradation complex located near the plasma membrane (Bienz, 1999).

Phenotypes have been reported for loss of *wg* function during pupal development (Shirras and Couso, 1996; Kopp et al., 1999).

Wg and Decapentaplegic (Dpp) are known to repress each other's expression during leg development (Morimura et al., 1996; Brook and Cohen, 1996). The mutually antagonistic interactions between Wg and Dpp ensure the maintenance of their expression in spatially restricted domains. The combined action of these two genes activates *Distal-less* (*Dll*) expression in the central region of the leg disc overlapping the anterior-posterior compartment boundary (Diaz-Benjumea et al., 1994). Dll is required for the proper specification of leg structures along the proximal-distal axis (Gorfinkiel et al., 1997; Campbell and Tomlinson, 1998).

Altered expression patterns of Wg and Dpp (Brook and Cohen, 1996) and phenotypes induced by loss of Wg (Held et al., 1997) are observed. Also, morphogenetic furrow progression (Ma et al., 1993, Treisman and Rubin, 1995) is documented. Dpp causing inhibition of morphogenetic furrow progression, which in turn leads to loss of ommatidia (Treisman and Rubin, 1995).

Similar leg and eye phenotypes were also induced by the intra-cellular domain of *Drosophila* E-cadherin (UAS-cadⁱ⁵ ) ; ( Sanson et al., 1996). Human APC could alleviate the effects of higher levels of β-catenin/Arm, when co-expressed with Flu-ΔArm (Zecca et al., 1996).

Increase in eye pigmentation and absence of pseudopupil is an indication of degeneration of retinal neurons (Ahmed et al. 1998).

Recently, it has been reported that all of the C-terminal domains of APC are dispensable in mouse. A mouse model homozygous for *APC1638T*, expressing a truncated protein with ability to bind β-catenin, is viable and tumor-free (Smits et al., 1999). This was supported by the observation that expression of the β-catenin binding domain of human APC alone was enough to down regulate β-catenin activity in colon cancer cell lines (Shih et al., 2000).

All the amino-acids (Arginine 386, Lysine 345 and Tryptophan 383 of human β-catenin; (Von Kries et al. 2000) in β-catenin that are associated with APC binding are conserved between *Drosophila* and human β-catenins (See "SEQ comparison 1" below).

Genetic studies described here on the *in vivo* function of APC have direct relevance in understanding the genesis of certain cancer types. For example, particular melanoma cell line, namely 888 mel, carries a single amino acid mutation, Ser³⁷ to Phe³⁷ substitution, in the N-terminal region of β-catenin.

Over-expression of APC in 888 mel cells does not down regulate β-catenin levels, suggesting that the mutant β-catenin is resistant to degradation. Interestingly, the mutant β-catenin is found accumulated on the APC protein and the two are localized near the plasma membrane (Rubinfeld et al., 1997).

In addition, it has been observed that even the over-expressed APC immuno-precipitates with β-catenin from 888 mel cells. This observation has been the basis for some speculation on the roles of β-catenin, one of them being down regulation of an unknown growth control function of APC (Polakis, 1997).

In the background of above results, it is plausible that in 888 mel cells both endogenous and over-expressed APC bind the mutant β-catenin with the same efficiency with which they bind wild type β-catenin. Since the mutant β-catenin is resistant to degradation due to the Ser³⁷ to Phe³⁷ substitution, the mutant β-catenin sequesters APC by inhibiting its reuse: all the endogenous APC would remain bound to the mutant β-catenin.

Constitutive activation of Wnt/Wg signaling would then be achieved by the excess β-catenin (both wildtype and mutant forms). Prediction from this model would be, although over-expressed APC does not down regulate β-catenin levels, it would still inhibit β-catenin activity. 888 mel cells also show changed cell-adhesion properties (Polakis, 1997).

Since β-catenin bound APC resides in the extensions of the plasma membrane (Nathke et al., 1996; Rubinfeld, 1997, Polakis, 1997; Bienz, 1999; this report), some amount of degradation-resistant β-catenin may ultimately reach the adhesion zones and thereby cause changes in the cell-adhesion properties. This can be attributed to the relative affinities of E-cadherin and APC to bind β-catenin/Arm: E-cadherin binds to β-catenin/Arm with higher affinity than APC.

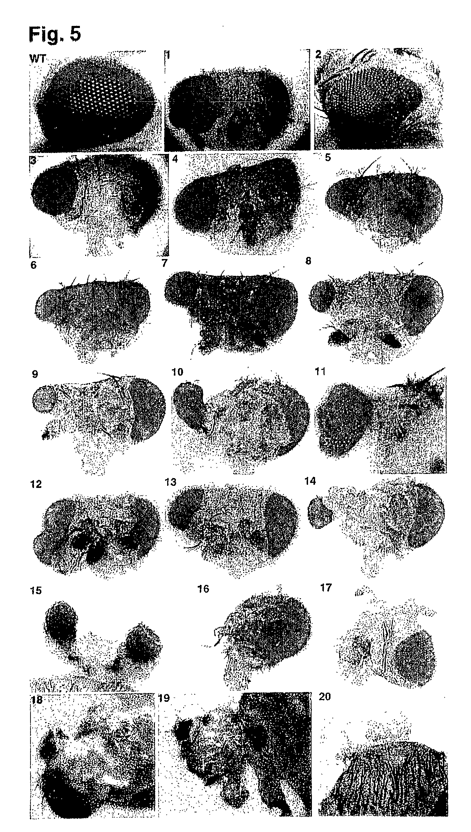

Recently, this classical genetic approach has been used to identify genes that modulated the phenotypes induced by the ectopically expressed human p53 in flies (Yamaguchi et al., 1999). UAS-hAPC/CBD was crossed to *ey*-GAL4 in the presence of additional mutations in the genome (only the 3^{rd} chromosome mutations were tested). It was observed that a deletion in the polytene region 85F (uncovered by *Df(3R)by62* and not by its overlapping deficiency *Df(3R)by10*) enhanced the eye phenotypes induced by human APC to such an extent that many dies were totally eyeless (Fig. 5; Table 1).

*Twins* (tws) is one of the many genes mapped to 85F region (http://flybase.bio.indiana.edu), which codes for the regulatory sub-unit of protein phosphatase 2A (PP2A). Biochemical experiments have implicated a role for PP2A in stabilizing β-catenin in the cytoplasm.

However, none of the three alleles of *tws* (*P1568*, *tws*^{*55*} *and tws*^{*60*}) that were tested enhanced human APC-induced eye phenotypes. Alleles of other neighbouring genes such as Ras (*Ras*^{*elB*}) and many of the available lethal P-insertions (P237, P1595, P1659 and P1783) mapping to 85F region (http://flybase.bio.indiana.edu) were also tried. None enhanced the human APC-induced eye phenotypes. This suggests the possibility of a hitherto unknown component of Wg signaling pathway in 85F region. Currently, EMS- and P-element induced mutations are being used to identify the gene.

This gene is being characterized and shows enhancement of phenotypes of certain *Wg* alleles such as *Wg*^{*P*} and *Spd*^{*flg*}, *Spd*^{*hL2*} (R Bajpai and LSS, unpublished observations).

APC anchors to different parts of the cell for efficient transport of β-catenin from the cytoplasm to the plasma membrane (Neufeld and White, 1997; Yu et al., 1999; Bienz, 1999; McCartney and Peifer, 2000). Consistent with this, in the intestine, APC is present at higher levels in the crypt/villus boundary (wherein larger amounts of β-catenin is released from adhesion complexes; Nathke et al., 1996). It is interesting to note here that invertebrate homologues of APC have much simpler secondary structure than their vertebrate counterparts

Wnt/Wg signaling stabilizes human APC/β-catenin/Arm complex.(Papkoff et al., 1996).

It has been shown that β-catenin/Arm degradation complex, consisting of dAPC2 and Axin, is formed in the epithelial cells near the apicolateral adhesive zones (McCartney et al., 1999; Yu et al., 1999). Human APC is also mainly localized near the plasma membrane (Nathke et al., 1999). It has been proposed that APC-dependent anchoring of the β-catenin/Arm degradation complex near the adhesion zones might enable cells to degrade free β-catenin/Arm more efficiently (Bienz, 1999

Due to N-terminal deletions UAS-S10 and Flu-ΔArm would not be phosphorylated by whatever the residual GSK-3β is present (Zecca et al, 1996; Pai et al., 1997).

It has been reported earlier that over-expression of Wnt-1 (which in turn inhibits GSK-3 β activity) in mouse mammary epithelial or pituitary cell lines is known to cause increased stabilization of APC/β-catenin complex (Papkoff et al., 1996). It has also been shown that in human cell lines, APC efficiently binds degradation-resistant forms of β-catenin (Rubinfeld et al., 1996). Degradation of β-catenin may help the cells to reuse APC protein (Bienz, 1999).

Attenuated Polyposis or nonpolyposis phenotypes are documented (Scott et al., 1996; Friedl et al., 1996).

Indomethacin is a non-steroid anti-inflammatory drug (NSAID) and affects the transcription
downstream of peroxisome proliferates activated receptor -type δ (PARδ); (He et al., 1999), a member of nuclear hormone receptors.

### Object of the present invention

The main object of the present invention is to develop an assay system using regulated mis-expression of the human Adenomatous Polyposis Coli (APC) gene in *Drosophila.*

Another object of the present invention is to use the said assay system to develop preventive and therapeutic drugs.

Yet another object of the invention is to use the said system tq determine the modulation and differential expression of the several APC interacting genes.

Still another object of the present invention is to determine the new target proteins interacting with β-catenin.

Still another object of the present invention is to understand the kinetics of Wnt/Wg signal transduction in *Drosophila.*

Still another object of the present invention is to study biochemical function of human APC function.

Still another object of the present invention is identify additional components of *Drosophila* Wnt/Wt signaling pathway.

### Summary of the present invention

The present invention relates to the development of an *in vivo* assay system. The said system is useful for developing therapeutic options. The drugs and other substances are tested for their efficacy using said system. The said system constitutes a novel, fast, and inexpensive *in vivo* assay system. The said system is consisting of transgenic *Drosophila* harboring human Adenomatous Polyposis Coli gene, a negative regulator of β-catenin.

### Detailed description of the present invention

Accordingly the present invention relates to a transgenic *Drosophila* whose genome comprises the full-length human colon cancer gene *Adenomatous Polyposis Coli* (APC) having SEQ ID NO.1 wherein:
(a) said genomic alteration allows mis-expression of full-length human APC in flies in regulated manner,
(b) said mis-expression of the full-length human APC results in developmental abnormalities,
(c) said developmental abnormalities induced by the mis-expression of full-length human APC in flies are similar to those exhibited by flies carrying mutations in *Drosophila wingless* gene, and
(d) to use the same as an assay system for screening and validating efficacy of drugs.

In an embodiment of the present invention the transgenic *Drosophila* genome includes β-catenin binding domain comprising of amino-acids from 959 to 1870 of SEQ ID NO. 2 from the full length human APC gene of SEQ ID NO.1, and this engineered disruption of human APC comprises only the five of the seven β-catenin binding domains wherein:
(a) said genomic alteration allows mis-expression of a truncated version of human APC in flies in a regulated manner,
(b) said mis-expression of the said gene construct results in the developmental abnormalities,
(c) said developmental abnormalities induced by the mis-expression of the said gene construct in flies is similar to those exhibited by flies carrying mutations in *Drosophila wingless* gene,
(d) said mis-expression of the said novel construct in regulated manner results in a more severe developmental phenotype, and
(e) to use the same as an assay system for screening and validating efficacy of drugs.

In another embodiment of the present invention, transgenic *Drosophila* has the N terminal domain of APC with amino acids from 1 to 767 having SEQ ID NO. 3, from the full length human APC gene of SEQ ID NO.1, wherein:
(a) said genomic alteration allows mis-expression of human APC in flies in a regulated manner,
(b) said mis-expression of the said novel construct in a regulated manner resulting in severe abnormalities in fly development during metamorphosis, and
(c) to use the same as an assay system for screening and validating efficacy of drugs.

In yet another embodiment of the present invention, a method for selecting a compound for pharmacological activity, which potentially inhibits or enhances the developmental abnormalities induced by the expression of full length and protein domains of human APC in *Drosophila*, said method comprising:
(a) providing the above-mentioned first, second, and third transgenic fly wherein said flies have said developmental abnormalities,
(b) administering the said compounds to the said transgenic *Drosophila* at different concentrations, and
(c) screening for the changes in the severity of the phenotype .

In still another embodiment of the present invention, a method of determining various *Drosophila* proteins interacting with full-length and protein domains human APC protein wherein, said method comprising:
(a) providing the above-mentioned first, second, and third transgenic fly, wherein said flies have said developmental abnormalities,
(b) crossing the said transgenic flies individually to a set of *Drosophila* strains each of which carries mutation in a different gene or set of genes, and
(c) Screening for the change in the severity of the phenotype.

In still another embodiment of the present invention, a method for determining the modulation and differential expression of genes following the mis-expression of full-length and its protein domains human APC in *Drosophila* wherein, said method comprising:
(a) providing the above-mentioned transgenic *Drosophila* wherein, the flies have . developmental abnormalities,
(b) screening for differential gene expression using differential display-RT PCR or microarray techniques, and
(c) identifying genes that are differentially regulated on expression of human APC.

In still another embodiment of the present invention, a method for determining the modulation and differential expression of proteins following the mis-expression of full-length and its protein domain human APC in *Drosophila* wherein, said method comprising:
(a) providing the above-mentioned transgenic *Drosophila* the flies have developmental abnormalities,
(b) identifying differential gene expression and protein modifications using proteomics techniques, and
(c) identifying gene products that are differentially regulated on expression of human APC.

In still another embodiment of the present invention, a method to study Wnt/Wg signaling in *Drosophila* said method comprising;
(a) providing the above-mentioned transgenic *Drosophila,*
(b) crossing these transgenic flies to a number of GAL4 drivers to induce targeted expression of said constructs in various tissues and at different developmental stages, and
(c) examining developmental abnormalities.

In still another embodiment of the present invention, methods wherein, examination of developmental abnormalities using gain-of-function genetic model for human APC to study mechanism of various developmental processes such as wing, leg, eye, antennae, and adult cuticle development.

In still another embodiment of the present invention, a method wherein, screening and validating efficacy of preventive and therapeutic drugs following APC gene mis-expression.

In still another embodiment of the present invention, a method wherein, human APC pathway is identified using drug selected from a group of compunds comprising anti inflammatory, Analgesics, Antipyretics, and Antineoplastics.

In still another embodiment of the present invention, a method wherein, concentration of said drugs ranging between 50 to 500 µg/ml of fly food.

In still another embodiment of the present invention, methods wherein, examination of developmental abnormalities using gain-of-function genetic model for human APC which has advantages to study the *Drosophila* Wnt/Wg signaling pathway.

In still another embodiment of the present invention, a method wherein, studying the kinetics of Wnt/Wg signaling during various developmental stages and in different tissues.

In still another embodiment of the present invention, a method wherein, new target proteins interacting with β-catenin are identified.

In still another embodiment of the present invention, a method wherein, genes interacting with APC are identified.

In still another embodiment of the present invention, methods wherein, examination of developmental abnormalities using gain-of-function genetic model for human APC to study biochemical function of human APC function.

In still another embodiment of the present invention, methods wherein, examination of developmental abnormalities using gain-of-function genetic model for human APC to identify additional components of *Drosophila* Wnt/Wg signaling pathway.

In still another embodiment of the present invention, a transgenic *Drosophila* whose genome comprises the full-length human colon cancer gene *Adenomatous Polyposis Coli* (APC) having SEQ ID NO. 1 wherein:
(a) said genomic alteration allows mis-expression of full-length human APC in flies in regulated manner,
(b) said mis-expression of the full-length human APC results in developmental abnormalities,
(c) said developmental abnormalities induced by the mis-expression of full-length human APC in flies are similar to those exhibited by flies carrying mutations in *Drosophila wingless* gene, and
(d) to use the same as an assay system for screening and validating efficacy of anti-cancer drugs.

In still another embodiment of the present invention, the transgenic *Drosophila* wherein, its genome includes β-catenin binding domain comprising of amino-acids from 959 to 1870 of SEQ ID NO. 2 from the full length human APC gene of SEQ ID NO.1, and this engineered disruption of human APC comprises only the five of the seven β-catenin binding domains wherein:
(a) said genomic alteration allows mis-expression of a truncated version of human APC in flies in a regulated manner,
(b) the mis-expression of the said gene construct results in the developmental abnormalities,
(c) the developmental abnormalities induced by the mis-expression of the said gene construct in flies is similar to those exhibited by flies carrying mutations in *Drosophila wingless* gene,
(d) mis-expression of the said novel construct in regulated manner results in a more severe developmental phenotype, and
(e) to use the same as an assay system for screening and validating efficacy of anti-cancer drugs.

In still another embodiment of the present invention, transgenic *Drosophila* wherein, the N terminal domain of APC with amino acids from 1 to 767 having SEQ ID NO. 3, from the full length human APC gene of SEQ ID NO.1 wherein:
(a) the said genomic alteration allows mis-expression of human APC in flies in a regulated manner,
(b) the mis-expression of the said novel construct in a regulated manner resulting in severe abnormalities in fly development during metamorphosis, and
(c) to use the same as an assay system for screening and validating efficacy of anti-cancer drugs.

In still another embodiment of the present invention, a method for selecting a compound for anti-cancer activity, which potentially inhibits or enhances the developmental abnormalities induced by the expression of full length and protein domains of human APC in *Drosophila*, said method comprising:
(a) providing the first, second, and third transgenic fly, wherein said flies have said developmental abnormalities,
(b) administering the said compounds to the said transgenic *Drosophila* at different concentrations, and
(c) screening for the change in the severity of the phenotype .

In still another embodiment of the present invention, a method wherein, screening and validating efficacy of anti-cancer drugs following APC gene mis-expression.

In still another embodiment of the present invention, a method wherein, human APC pathway is identified using drugs selected from a group of anti-cancer compounds comprising anti inflammatory, Analgesics, Antipyretics, and Antineoplastics.

In still another embodiment of the present invention, a method wherein, concentration of said anti-cancer drugs ranging between 50 to 500 µg/ml of fly food.

In yet another embodiment of the present invention, a transgenic non-human animal expressing human colon cancer gene, Adenomatous polyposis Coli (*APC*). Applicants have generated transgenic flies that express human APC, an important tumor suppressor gene, and mutations in which cause both familial and sporadic colorectal cancers.

In still another embodiment of the present invention, transgenic *Drosophila* expressing either full length or truncated forms of human APC protein and methods for using it as an assay system for identification and development of preventive as well as therapeutic dings. against cancer and other disease conditions.

In still another embodiment of the present invention, consistent with its biochemical properties, targeted expression of either full-length APC or its truncated form negatively regulate the function of the β-catenin homologue, Armadillo (Arm). This in turn results in the inhibition of Wnt/Wg signaling during fly development and give rise to phenotypes similar to those associated with loss of Wnt/Wg signaling in *Drosophila*.

In still another embodiment of the present invention, the transgenic fly of the invention can be used to study genetic and biochemical pathways associated with colorectal and other cancers.

In still another embodiment of the present invention, the phenotypes generated in the fly due to the mis-expression of human APC would constitute a novel, fast and less expensive *in vivo* assay system for the identification of targets for drug screening and validation of vast repertoire of drugs against cancer and other disease conditions.

### List of the accompanying drawings:

**Fig. 1** shows human APC constructs used for generating transgenic flies and phenotypes induced by their expression in *Drosophila*.
**Fig. 2** shows human APC suppression phenotypes, induced by elevated levels of β-catenin/Arm and rescues phenotypes associated with mutant *dAPC*.
**Fig. 3** shows human APC induced changes in the levels of β-catenin/Arm in different cell types.
**Fig. 4** shows effect of GSK-3 β/Sgg activity on the interaction between human APC and β-catenin/Arm.
**Fig. 5** shows enhancement of human APC induced eye phenotypes in *Drosophila.*

**Table 1** Distribution of eye phenotypes induced by hAPC/CBD in different genetic backgrounds and in the presence of various compounds and other anti-cancer drugs. Numbers are from a representative experiment. Number in parenthesis in the last column indicates total number of the flies of right genotype.

| **Phenotypic ranks** | | | | | |
|---|---|---|---|---|---|
| **Genotypes** | **1-5** | **6-10** | **11-15** | **16-20** | **Total** |
| Ey-GAL4;hAPC/CBD | 13 | 5 | - | - | 18(49) |
| Ey-AL4;hAPC/CBD/Df(3R)by⁶² | 10 | 7 | 12 | 9 | 38(86) |
| Ey-GAL4;hAPC/CBD/1(3L)67E2 | 7 | 6 | 6 | 5 | 24(63) |
| Ey-GAL4; hAPC/CBD in the presence of 5-fluorouracil at 50 micrograms/ml fly food. | 9 | 4 | 1 | - | 14(47) |
| Ey-GAL4; hAPC/CBD in the presence of Indomethacin at 200 microgram/ml fly food. | 12 | 8 | 16 | 11 | 47(121) |
| Ey-GAL4; hAPC/CBD in the presence of aspirin at 200micrograms/ml fly food. | 13 | 4 | 2 | - | 19(61) |
| Ey-GAL4; hAPC/CBD in the presence of Pyroxicam at 500 micrograms/ml fly food. | 11 | 7 | 1 | 2 | 21(59) |

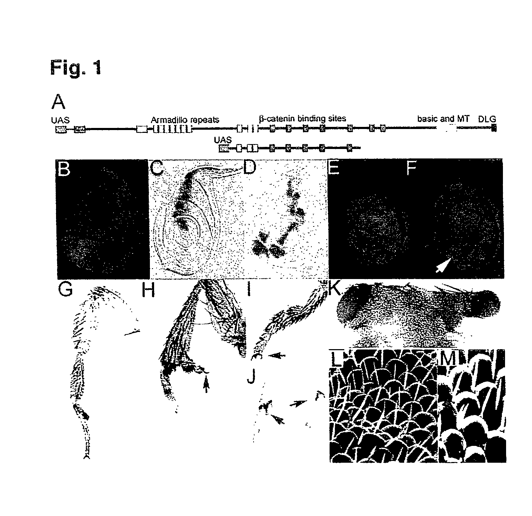

In an embodiment of the present invention, transgenic flies are generated for the full-length human APC (hAPC/FL) as well as for the β-catenin binding domain (hAPC/CBD) and the N-terminal domain (hAPC/NBD) (Fig. 1*A*; Example 1).

In another embodiment of the present invention, CAL4-UAS system is employed for targeted expression of human APC constructs in different tissues during development and in a number of genetic backgrounds.

In yet another embodiment of the present invention, alternative expression systems such as those, which employ heat-shock or tissue-specific promoters are also available which can also be used for expression of genes of interest. These systems may also be effective in giving phenotype with human APC.

In still another embodiment of the present invention, both full-length and the β-catenin binding domain give comparable phenotypes, although levels of expression of full-length protein are lower than those of the β-catenin binding domain.

In still another embodiment of the present invention, effects of hAPC/FL expression are less severe than hAPC/CBD. Wherever both hAPC/FL and hAPC/CBD give comparable phenotypes, the term human APC is used to refer to them together. Mis-expression of hAPC/NBD resulted in severe defects in metamorphosis and head involution.

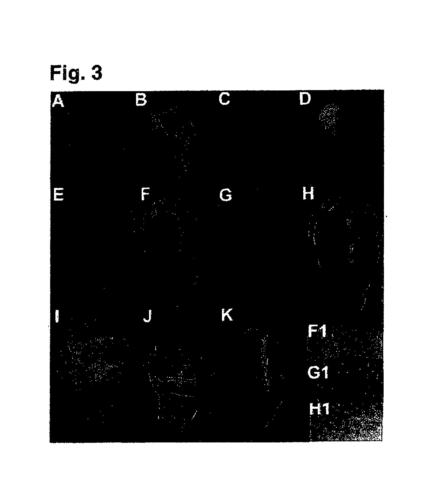

In still another embodiment of the present invention, expression of human APC in flies was confirmed by both RNA in situ (data not shown) and antibody staining (Fig. 3*B*, *F*). It was first ensured that behavior of APC in flies is consistent with its biochemical role in human.

In an embodiment of the present invention, to determine if human APC interferes with Wingless (Wnt/Wg) signaling in flies, its expression is targeted to Wg-dependent developmental pathways.

In another embodiment of the present invention, UAS-hAPC/FL and UAS-hAPC/CBD are mis-expressed in different tissues such as epidermis, mesoderm, central and peripheral nervous systems and endoderm and at different developmental time stages with the help of a number of tissue-specific GAL4-driver lines.

In yet another embodiment of the present invention, both the above-mentioned constructs induce phenotypes in adult legs, wings, antennae and eyes. In addition, the full-length construct, when expressed with ptc-GAL4 driver, affected adult epidermal development. The dorsal tergites are not properly developed and were devoid of pigmentation (Fig. 3B).

In still another embodiment of the present invention, expression of human APC in leg discs using *ptc-GAL4* resulted in ectopic expression of Dpp (Fig. 1*D*), suggesting negative regulation of Wnt/Wg signaling.

In still another embodiment of the present invention, ectopic Distal-less (Dll) expression is also seen (Fig. 1*F*), which reflects altered expression patterns of Wg and Dpp.

In still another embodiment of the present invention, adult flies expressing human APC exhibited leg phenotypes such as leg duplications (Fig. 1*H-J*) that are similar to the phenotypes induced by loss of Wg.

In still another embodiment of the present invention, in developing eye imaginal discs too, Wg negatively interact with Dpp to regulate the morphogenetic furrow progression. Gain of Dpp or loss of Wg results in the activation of retinal differentiation along dorsal and ventral margins. This leads to inappropriate patterning of ommatidia, leading to either ectopic ommatidia in the anterior head capsule or in severe cases. loss of ommatidia.

In still another embodiment of the present invention, expression of human APC in the eye disc using *ey*-GAL4 resulted in ectopic ommatidia resulting in the protrusion of the eye over the anterior head capsule (Fig. 1*K-M*). The phenotypes are identical to those induced by the over-expression of Dpp or activated form of its receptor Thick-vein, further suggesting down regulation of Wnt/Wg signaling by human APC.

In still another embodiment of the present invention, in addition, human APC-induced phenotypes in many other appendages/tissues such as wing, antennae and adult cuticle all of which resemble phenotypes caused by reduced Wnt/Wg signaling. These observations reveal that human APC expression induced specific phenotypes, which mimic loss of Wg signaling.

In an embodiment of the present invention, similar leg and eye phenotypes are also induced by the intra-cellular domain of *Drosophila* E-cadherin (UAS-cadⁱ⁵), which is known to deplete the cytoplasmic pool of Armadillo (β-catenin/Arm).

In another embodiment of the present invention, since APC is a known β-catenin-binding protein, it is hypothesized that human APC-induced phenotypes are also mediated by the negative regulation of β-catenin/Arm. This is supported by the enhanced leg phenotypes (often leading to total loss of appendage development) induced by the expression of human APC using *ptc-*GAL4 in flies heterozygous for a null mutation in *arm.*

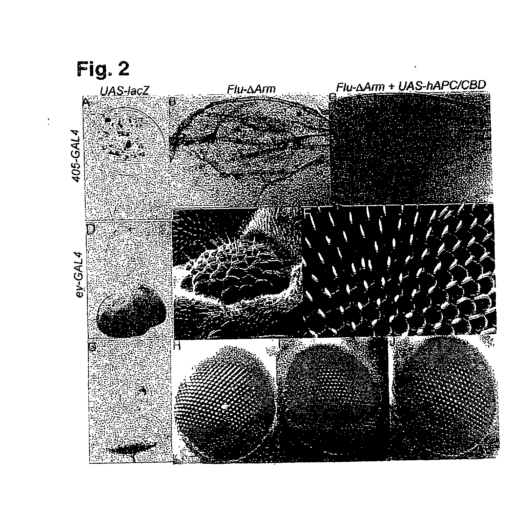

In yet another embodiment of the present invention, in addition, human APC could alleviate the effects of higher levels of β-catenin/Arm, when co-expressed with Flu-ΔArm, a stable form of β-catenin/Arm (Fig. 2*C*).

In still another embodiment of the present invention, finally, possibility of suppression of human APC-induced phenotypes by the over-expression of β-catenin/Arm is examined. Enhanced Wg activity in developing eye imaginal discs down-regulates Dpp causing inhibition of morphogenetic furrow progression, which in turn leads to loss of ommatidia. Consistent with this, mis-expression of Flu-ΔArm using *ey*-GAL4 result in severe loss of ommatidia. Although hAPC/CBD (Fig. 1*K-M*) and Flu-ΔArm (Fig. 2*E*) induce eye abnormalities when expressed individually using *ey*-GAL4, flies expressing both had normal eyes (Fig. 2*F*).

In still another embodiment of the present invention, this suggests that, at least in the eye, human APC-induced phenotypes are entirely due to its interaction with β-catenin/Arm.

In still another embodiment of the present invention, these observations also suggest that human APC expression in the heterologous system does not lead to any dominant-negative effect.

In still another embodiment of the present invention, applicants observe that the down regulation of β-catenin/Arm activity in the above mentioned experiments is not a mere reflection of the presence of overwhelming amount of human APC, since human APC could also alleviate dominant phenotypes caused by the over-expression of β-catenin/Arm.

In'still another embodiment of the present invention, to further validate genetic studies on hAPC in this heterologous system, it is further determined whether *hAPC* could be substituted for its *Drosophila* homologue *dAPC*.

In still another embodiment of the present invention, *dAPC*^{*Q8*}, a viable allele of *dAPC* in which all surviving homozygous flies show increase in eye pigmentation and absence of pseudopupil, an indication of degeneration of retinal neurons (Fig. 2*I*) is used in the above mentioned experiment. hAPC/FL is expressed in *dAPC*^{*Q8*} / *dAPC*^{*Q8*} background with the help of 405-GAL4 driver, which is expressed in the differentiating retinal neurons (Fig. 2*G*). All the flies expressing hAPC/FL in *dAPC*^{*Q8*} / *dAPC*^{*Q8*} background show the presence of pseudopupil and normal levels of eye pigmentation suggesting complementation of *dAPC* by hAPC/FL (Fig. 2*J*).

In still another embodiment of the present invention, applicants have shown that human APC-induced phenotypes in flies are consistent with the role of human APC as a negative regulator of β-catenin/Arm activity thereby, supporting the use of fly model system for genetic analyses of human APC function.

In still another embodiment of the present invention, however, UAS-dAPC1 induce only very weak phenotypes when expressed using various GAL4 lines and at different temperature conditions. Its ability to suppress the phenotypes induced by over-expression of dominant forms of β-catein/Arm is also negligible. This is particularly intriguing considering the specificity of human APC-induced phenotypes in flies. One possible explanation is, human APC has higher affinity for Arm than dAPC1 or EAPC. This is supported by the fact that β-catenin is far more highly conserved than APC across a number of species from *Drosophila* to human. For example all the amino-acids (Arginine 386, Lysine 345 and Tryptophan 383 of human β-catenin; in β-catenin that are associated with APC binding are conserved between *Drosophila* and human β-catenins (See "SEQ comparison 1" below).

In still another embodiment of the present invention, hAPC/CBD alone is enough to bind and transport β-catenin to degradation zone and to subject the same for degradation in the presence of GSK-3 β activity. Interestingly, hAPC/CBD, which comprises only the five of the seven β-catenin binding domains result in far more severe developmental phenotypes than hAPC/FL. This may reflect enhanced ability of human APC to bind β-catenin/Arm due to the engineered disruption of the protein.

In still another embodiment of the present invention, expression of human APC result in stronger phenotypes indicates the uniqueness of this transgenic system to study genetic and biochemical pathways related to APC function.

In an embodiment of the present invention, the nature of human APC-induced adult phenotypes and its ability to suppress the effects of degradation-resistant Flu-ΔArm suggest down-regulation or sequestration of β-catenin/Arm in human APC-expressing cells. Therefore, changes in the levels of β-catenin/Arm following human APC expression were monitored.

In another embodiment of the present invention, normally, β-catenin/Arm is present in all the cells, although its levels are slightly higher in cells where Wnt/Wg signaling is active (for example, in the wing disc D/V boundary; Fig. 3*G*). It is observed that a subset of human APC-expressing cells exhibited enhanced levels of β-catenin/Arm (Fig. 3*D*, *H*, *J*). In other cells, the levels of β-catenin/Arm are comparable to the wildtype levels.

In yet another embodiment of the present invention, double staining for β-catenin/Arm and Wg reveal that β-catenin/Arm is accumulated in cells where human APC and endogenous Wg expression overlapped (Fig. 3*I*). Wnt/Wg signaling inhibits the degradation of β-catenin/Arm by down regulating the activities of GSK-3 β/Sgg and Axin (7). This results in the increased levels of cytoplasmic β-catenin/Arm.

In still another embodiment of the present invention, accumulation of β-catenin/Arm only in cells expressing both human APC and endogenous Wg suggests that (i) human APC can efficiently inhibit the function of cytoplasmic β-catenin/Arm even in the cells where Shaggy/Zeste-White3 (GSK-3 β/Sgg) activity is down regulated by Wnt/Wg signaling and (ii) human APC-induced phenotypes are due to the sequestration of β-catenin/Arm.

In still another embodiment of the present invention, immuno-precipitation and Western blot analyses does not show any marked increase in the levels of β-catenin/Arm over the control. This is due to the fact that human APC-mediated accumulation of β-catenin/Arm is seen only in a small fraction of cells.

In still another embodiment of the present invention, since human APC can efficiently bind and sequester larger amounts of β-catenin/Arm in cells responding to Wnt/Wg signaling, it is presumed that in other cells too it can bind β-catenin/Arm. It implies that in those cells either human APC-bound form of β-catenin/Arm is recognized by the Axin-GSK-3 β/Sgg complex for degradation or no free cytoplasmic β-catenin/Arm is available for human APC to bind. The latter possibility is ruled out since ectopic expression of UAS-cad¹⁵ resulted in the accumulation of large and equal amounts of β-catenin/Arm in all the cells, irrespective of the presence or absence of Wnt/Wg signaling (Fig. 3*K*).

In still another embodiment of the present invention, the regulation of β-catenin/Arm at the transcriptional level using *arm*-lacZ construct is examined. There is no change in lacZ levels in response to human APC expression in different tissues. This suggests that human APC-induced increase in the levels of β-catenin/Arm protein (Fig. 3*D*, *H*, *J*) is due to stabilization of human APC/β-catenin/Arm complex by Wnt/Wg signaling.

In still another embodiment of the present invention, the development of a gain-of-function genetic model for human APC similar to the one described here, has certain advantages to study Wnt/Wg signaling in *Drosophila*.

In still another embodiment of the present invention, the role of β-catenin/Arm in cell-adhesion has always been a limitation to generate loss-of-function clones of *arm* cells to study Wnt/Wg signaling during post-embryonic stages. Such clones are invariably cell-lethal due to loss of cell-adhesion and do not produce any data on Wnt/Wg signaling events.

In still another embodiment of the present invention, it has been demonstrated that mis-expression of human APC down-regulate Wnt/Wg signaling by binding to cytoplasmic β-catenin/Arm, thus enabling one to study the kinetics of Wnt/Wg signaling during development.

In still another embodiment of the present invention, applicants have examined the expression patterns of a number of limb patterning genes (such as *dpp*, *wg*, *Dll*, *dauschund*, *cut*, etc.) by down-regulating Wnt/Wg signaling at different developmental stages and in different regions of the developing imaginal discs.

In still another embodiment of the present invention, applicants have also studied the requirement for Wnt/Wg signaling in different tissues and developmental stages by inhibiting β-catenin/Arm activity.

In an embodiment of the present invention, in transgenic flies expressing human APC, both human APC and accumulated β-catenin/Arm are localized near the plasma membrane (Fig. *3C1*, *F1*, *H1*).

In another embodiment of the present invention, even in the absence of any detectable accumulation of β-catenin/Arm i.e. in cells where GSK-3β/Sgg is active, much of the human APC is localized near the plasma membrane.

In yet another embodiment of the present invention, similar sub-cellular distribution of human APC in both kinds of cells, i.e. cells responding to Wnt/Wg signaling and otherwise, is an additional evidence to show that human APC can not only bind *Drosophila* Arm, but also can transport it to the plasma membrane for degradation.

In an embodiment of the present invention, the requirement of GSK-3β/Sgg activity for human APC-mediated inhibition of β-catenin/Arm function was examined. hAPC/CBD and the degradation-resistant form of Arm (UAS-S10 or Flu-ΔArm) are co-expressed in the wing disc D/V boundary using *vg-* GAL4.

In another embodiment of the present invention, in the wing disc D/V boundary both Wg (Fig. 3*G*) and β-catenin/Arm (Fig. 3*C*) are maximally expressed and GSK3β/Sgg activity is expected to be minimum. In addition, due to N-terminal deletions UAS-S10 and Flu-ΔArm would not be phosphorylated by whatever the residual GSK-3β is present.

In yet another embodiment of the present invention, both hAPC/FL and hAPC/CBD suppress the phenotypes induced by Flu-ΔArm and UAS-S 10 (data not shown) with the same efficiency as it suppressed the ectopic β-catenin/Arm activity in a non-Wg region (Fig. 2*C*).

In still another embodiment of the present invention, GSK-3β/Sgg activity is not essential for the binding of human APC to β-catenin/Arm and the subsequent inhibition of β-catenin/Arm function.

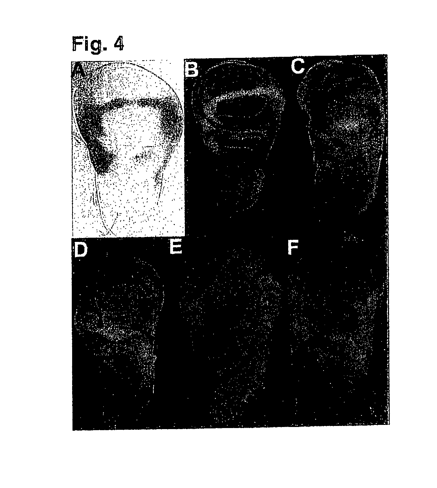

In still another embodiment of the present invention, to further examine the requirement of GSK-3β/Sgg activity for the human APC-mediated degradation of β-catenin/Arm, human APC and/or GSK-3β/Sgg are expressed in wing imaginal discs using *vg-*GAL4 driver. Expression of hAPC/CBD using *vg*-GAL4 resulted in the accumulation of large amount of β-catenin/Arm in the D/V boundary (Fig. 4*B*).

In still another embodiment of the present invention, whereas, over-expression of GSK-3 β/Sgg alone or together with hAPC/CBD result in reduced β-catenin/Arm levels in all the cells, which is more dramatic in the D/V boundary (Fig. 4*C*, *D;* compare with Fig. 3*H*).

In still another embodiment of the present invention, human APC binds to β-catenin/Arm in all the cells, although β-catenin/Arm is degraded only in cells where .GSK-3β/Sgg is active. However, human APC levels arc similar in all cells irrespective of the presence or absence of GSK-3β/Sgg activity (Fig. 4*E*). The levels of human APC are not altered even in the cells over-expressing GSK-3β/Sgg (Fig. 4*F*).

In still another embodiment of the present invention, thus, human APC is able to suppress with equal efficiency β-catenin/Arm-induced dominant phenotypes in the presence as well as in the absence of GSK-3β/Sgg activity.

In still another embodiment of the present invention, the uncoupling of inhibition of β-catenin activity from its degradation suggest that human APC inhibited Wnt/Wg signaling by sequestering β-catenin and not by the down regulation of its protein levels.

In still another embodiment of the present invention, the blockage of β-catenin degradation by Wnt signaling would affect the release of free APC from the degradation complex. Thus, in cells carrying gain-of-function mutations in Wnt or β-catenin, APC itself is sequestered by β-catenin leading to accumulation of free β-catenin in the cytoplasm, which would now be available to transduce Wnt signal.

In still another embodiment of the present invention, although the final outcome would be similar to what happens in *APC*⁻ colon cancer cells, the effects of loss-of-function mutations in APC are more pronounced in certain kinds of cells such as colonic crypts. Virtually all cells would be affected if the increased oncogenic activity of β-catenin is due to sequestration of APC.

In still another embodiment of the present invention, abolition of Axin and APC interaction would inhibit the degradation of β-catenin, but would not affect the β-catenin binding ability of APC. Although this would lead to increase in β-catenin levels, the phenotype would be less severe when compared to that of APC⁻ cells. Indeed, colorectal cancers caused by mutations in APC that delete only the Axin binding sites show either attenuated polyposis or nonpolyposis phenotypes.

In addition, mutations in 67E2 (*l(3)67E2* caused by the imprecise excision of a P-element insertion) also showed enhanced eye-phenotypes, although less severely compared to *Df(3R)by*^{*62*} (Table 1).

In an embodiment of the present invention, transgenic flies expressing human APC are tested for their usefulness in screening potential drugs against colon cancer.

In another embodiment of the present invention, as pilot experiments, some of the known anti-colon cancer drugs, for example, 5-fluorouracil, pyroxicam, indomethacin, and aspirin are tested for their effect on *Drosophila.*

In yet another embodiment of the present invention, LD50 is determined for each of the chemicals tested. At concentrations LD50 and below, none of the drugs induce any morphological phenotypes in wildtype flies. Thereafter, the effect of these drugs on transgenic flies expressing human APC in developing eyes (using *ey*-GAL4) is examined.

In still another embodiment of the present invention, Indomethacin enhances human APC-induced eye phenotypes to the extent similar to the enhancement shown by *Df(3R)by*^{*62*} (Fig. 5 and Table 1).

In still another embodiment of the present invention, the effect is concentration dependent, maximum being at 200 µg / ml of fly food (LD50 being at 250 µg/ml of fly food).

In an embodiment of the present invention, the protein sequence of PPARδ is compared to that of *Drosophila* proteins (known and predicted). Eip75B, a transcription factor in :ecdysone response pathway, shows 38%) identity and 59% homology to PPARδ, (however, Eip75B is 1443 amino acid long, whereas PPARδ is 441 amino acid long).

In another embodiment of the present invention, although Eip75B is not known to modulate Wg signaling, these results show the potential use of transgenic flies in identifying targets (and thereby mode of action) of drugs that are already being used in addition to their usefulness in identification of new target proteins.

In yet another embodiment of the present invention, applicants have developed an assay system that enables relatively easy and fast identification of suitable candidate genes for drug screening.

In still another embodiment of the present invention, this assay system allows the identification of molecular targets and biochemical pathways modulated by currently used cancer drugs, whose mechanism of action is not known.

In still another embodiment of the present invention, applicants have taken genetic approach to examine, *in vivo,* the interactions between APC, β-catenin and other components of Wnt/wg signaling pathway and the significance of the same in development and tumor formation. However, structure-function relationship studies of large multi-domain proteins, such as APC, require simple model systems amenable to various kinds of genetic and *in vivo* biochemical analyses

In still another embodiment of the present invention, applicants have generated flies transgenic for (i) the full-length human APC (ii) its N terminal fragment and (iii) for the fragment bearing only the β-catenin binding domains. These transgenic flies are used to correlate biochemical interactions amongst APC, β-catenin and GSK-3β to their *in vivo* function

In still another embodiment of the present invention, consistent with its biochemical properties, targeted expression of either full-length APC or its β-catenin binding domain alone negatively regulate the function of the β-catenin homologue, Armadillo (Arm) and thereby, inhibited Wnt/Wg signaling during fly development.

In still another embodiment of the present invention, although APC is a large multi-domain protein, the β-catenin binding domain of APC alone is sufficient to functionally complement mutations in its *Drosophila* homologue. APC inhibited Arm function even in the absence of GSK-3β activity, although the latter is required to mediate the degradation of Arm. Consistent with this, APC suppressed the phenotypes induced by the over-expression of degradation-resistant forms of Arm.

In still another embodiment of the present invention, interestingly, transgenic construct carrying only β-catenin binding domain of human APC, which comprises only the five of the seven β-catenin binding domains resulted in far more severe developmental phenotypes than full-length protein. This may reflect enhanced ability of human APC to bind β-catenin/Arm due to the engineered disruption of the protein.

In still another embodiment of the present invention, moreover, the phenotypes induced by over-expression of human APC in *Drosophila* are far more severe, although qualitatively similar, than those induced by *Drosophila* homologue of APC.

In still another embodiment of the present invention, thus, the observation that mis-expression of human APC, particularly the mis-expression of β-catenin binding domain alone, results in stronger phenotypes indicates the uniqueness of this transgenic system to study genetic and biochemical pathways related to APC function.

In still another embodiment of the present invention, applicants' results show that transgenic flies expressing human APC is not only a good model system, but also a unique system for genetic studies on APC function.

In still another embodiment of the present invention, applicants have identified two new loci in *Drosophila*, which act as positive regulators of Wnt/Wg signaling.

In still another embodiment of the present invention, the usefulness of assays based on human APC-induced phenotypes in *Drosophila* for the identification of targets for drug screening and validation of potential drugs against cancer and other diseases resulting due to high levels of β-catenin is also demonstrated.

In still another embodiment of the present invention, applicants have also confirmed that one of the currently used anti-cancer drugs specifically enhanced human APC-induced phenotypes thereby demonstrating that it is a specific inhibitor of Wnt signaling. This confirms the usefulness of the claimed genetic system for the screening of bioactive molecules effective against cancer and other disease conditions.

In still another embodiment of the present invention, the human APC induced phenotypes in *Drosophila* can be employed for studying the function of the human APC protein and for developing therapeutics for the prevention and treatment of cancer and other disease conditions caused by high levels of β-catenin.

### Brief description of the accompanying drawings

**Fig. 1** shows Human APC constructs used for generating transgenic flies and phenotypes induced by their expression in *Drosophila*. **(A)** Structure-function map of human APC full-length protein. MT- microtubule binding, DLG- Discs Large tumor-suppressor protein binding domains. Transgenic flies expressing full-length (hAPC/FL) and β-catenin binding domains (hAPC/CBD) have been reported here. They were expressed using either *ptc*-GAL4 (D, F-J) or *ey*-GAL4 (K-M). In all the leg discs shown in B-F, anterior is to the left and ventral is up. **(B)** Anti-Wg staining to show wildtype pattern of Wg expression in leg discs. Note that Wg is expressed only in the ventral-anterior compartment. **(C)** Expression pattern of *dpp*-lacZ reporter gene construct, in wildtype leg disc. Note that *dpp*-lacZ is expressed predominantly in the dorsal-anterior compartment. **(D)** hAPC/CBD expression results in the activation of dpp-lacZ in the ventral-anterior compartment. **(E)** Dll expression pattern in a wildtype leg disc as detected by anti-Dll staining **(F)** hAPC/CBD-induced ectopic Dll expression (arrow). **(G)** A wildtype leg. **(H-J)** human APC-induced leg phenotypes. They include branched legs (arrow in H), duplication of claws (arrow in 1) and sex-combs (arrow in J). **(K-M)** human APC-induced eye phenotypes. Often, the eyes were protruding anteriorly due to ectopic ommatidia on the head capsule. However, the total number of ommatidia was often less than normal (K). Human APC also induced duplication of inter-ommatidial bristles (L-M; arrow in M).

**Fig. 2** shows Human APC suppresses the phenotypes induced by elevated levels of β-catenin/Arm and rescues phenotypes associated with mutant *dAPC*, **(A)** Expression pattern of 405-GAL4 in wing discs as indicated by lacZ staining. **(B)** Flu-Δarm/405-GAL4 induced wing phenotype. Note large number of necrotic patches on the wing blade. **(C)** Rescue of wing phenotype in flies expressing both hAPC/CBD and Flu-Δarm. Expression of hAPC/CBD alone using 405-GAL4 did not induce any phenotype. **(D)** The expression pattern of *ey*-GAL4 in eye discs as indicated by lacZ staining. **(E)** Expression of Flu-Δarm in developing eye discs results in severe loss of ommatidia. **(F)** Co-expression of hAPC/CBD and Flu-Δarm restores normal organization of ommatidia. **(G)** The expression pattern of 405-GAL4 in eye discs as indicated by lacZ staining. This GAL4 was used to express hAPC/FL in differentiating retinal neurons. **(H)** Eye of a *dAPC*^{*Q8*} heterozygous fly. Note the presence of pseudopupil, a dark spot in the middle of the eye (asterisk). **(I)** Eye of a homozygous *dAPC*^{*Q8*} *fly.* This loss-of-function mutation in *dAPC* causes degeneration of retinal neurons, which is reflected in the absence of pseudopupil. Also note increase in eye pigmentation. **(J)** Rescue of eye phenotype in 405-GAL4; hAPC/FL *dAPC*^{*Q8*} / *dAPC*^{*Q8*} fly. Note normal pigmentation and re-appearance of pseudopupil.

**Fig. 3** shows Human APC induced changes in the levels of β-catenin/Arm in different cell types. The *ptc*-GAL4 was used to express hAPC/CBD (B, D, F, H), hAPC/FL (J) and UAS-Cadⁱ⁵ (K). (A, E) Anti-human APC staining of wildtype leg (A) and wing (E) imaginal discs showing the expression pattern of endogenous dAPC. (B, F) anti-human APC staining following hAPC/CBD expression in leg (B) and wing (F) discs. (C, G) anti-Arm staining of wildtype leg (C) and wing (G) discs. (D, H, J) anti-Arm staining following hAPC/CBD expression in leg (D) and wing (H) discs and hAPC/FL expression in wing discs (J). Note increased β-catenin/Arm levels. This was true for eye-antennal imaginal discs too. (I) Wing disc expressing hAPC/CBD stained with anti-Arm (green) and anti-Wg (red). Higher levels of β-catenin/Arm are seen only in Wg-expressing cells. (K) UAS-Cadⁱ⁵ expression induces uniform accumulation of β-catenin/Arm all along the A/P axis. (F1, G1, H1) Higher magnification images of F, G, H respectively, to show subcellular localization of β-catenin/Arm and human APC. In both wild-type cells (Gl) and in human APC-expressing cells (H1), β-catenin/Arm is predominantly localized near the plasma membrane. This has been confirmed by double staining with DAPI, which stains DNA (data not shown). Much of the human APC protein is also seen near the plasma membrane (F1). In all the wing and leg discs shown in this figure, anterior is to the left and ventral is up.

**Fig. 4** shows Effect of GSK-3 β/Sgg activity on the interaction between human APC and β-catenin/Arm. **(A)** The expression pattern of *vg*-GAL4 in wing discs as indicated by lacZ staining. This GAL4 driver was used to express hAPC/CBD **(B, D-F)** and/or GSK-3 β/Sgg **(C, D, F).** Discs in B-D are stained with anti-Arm and E, F for anti-human APC antibodies. (B) hAPC/CBD expression results in the accumulation of β-catenin/Arm, but only in the D/V boundary. Whereas, over-expression of GSK-3 β/Sgg (C) alone or with hAPC/CBD (D) results in the lowering of β-catenin/Arm levels in both D/V and non-D/V boundary cells. (E) hAPC/CBD expression as driven by vg-GAL4. (F) Wing disc co-expressing both hAPC/CBD and GSK-3 β/Sgg. human APC expression is robust as in E.

**Fig. 5** shows Enhancement of human APC induced eye phenotypes in *Drosophila* by two independent deletion mutations in the third chromosome and by the non-steroid anti-inflammatory drug, indomethacin. Eye phenotypes induced by human APC are arranged in the order of severity from 1 to 20. Human APC in wildtype background induced phenotypes of ranks 1-9. Expression of human APC in the presence of either *Df(3R)by*^{*62*} or *l(3L)67E2* or the drug indomethacin showed much enhanced phenotypes (rank 1-20) often resulting eyeless flies. WT - wild type eye.

The following examples are given by way of illustrations and therefore should not be construed to limit the scope of the present invention.

### EXAMPLES

### Example 1

Full-length human APC cDNA clone (Kinzler et al., 1991; SEQ ID No. 1) was released from its vector (pCMV) by BamH1 digestion. The 8.9kb insert was sub-cloned in to the P-element vector, pCaSpeR-UAS (Brand and Perrimon, 1993). The β-catenin binding domain (comprising of amino-acids from 959 to 1870; SEQ ID NO. 2) was released from a partial cDNA clone of human APC (FB10B; Kinzler et al., 1991) by EcoR1 and Xbal digestion. The 2.7kb fragment was independently sub-cloned into pCaSpeR-UAS. The N terminal domain of APC (comprising of amino-acids from 1 to 767; SEQ ID NO. 3) was released from full-length cDNA clone and was subcloned into pCaSpeR-UAS.

### Example 2

All the constructs were injected into *w*; Δ2-3 *Ki* embryos carrying a genetic source of transposase (Cooley et al., 1988). 15 independent transgenic lines for the full-length construct and 14 for the β-catenin binding domain were generated. UAS-dAPC1 was generated by Y Ahmed and E Wieschaus (unpublished) using cDNA clone for *dAPC1*, the first *Drosophila* homologue of *APC*. The X-chromosome insertion of UAS-dAPC1 was mobilized by crossing to a genetic source of transposase (w; Δ2-3 *Sb*/ Δ2-3 *TM2*).

### Example 3

All the transgenic flies were crossed to various GAL4 drivers to express human APC in different tissues. Recombinant chromosomes and combinations of other UAS lines, different mutations and/or markers were made by standard genetic techniques. Other UAS lines used were, UAS-Flu-ΔArm (N-terminal 155 residues are deleted in this construct due to which over-expressed Arm is resistant to GSK-3β-mediated degradation, but can displace endogenous β-catenin/Arm from the adhesion complex; Zecca et al., 1996), UAS-S10 (another activated form of Arm resistant to CSK-3β-mediated degradation due to an internal deletion of N-terminal 43-87 residues, but present both in the nucleus and cytoplasm; Pai et al., 1997), UAS-Zw3 (GSK-3β/Sgg; Steitz et al., 1998), UAS-Cadⁱ⁵ (intracellular domain of *Drosophila* E-cadherin; Sanson et al., 1996), UAS-Dpp (Frasch, 1995) and UAS-activated Tkv (Thick-vein a receptor of Dpp; Nellen et al., 1994). GAL4 strains used were *ptc-* and *en*-GAL4 (Brand and Perrimon, 1993), *dpp*-CAL4 (Morimura et al., 1996), *ey*-GAL4 (Hazelett et al., 1998), *vg*-GAL4 (Simmonds et al., 1995) and 405-GALA (LSS, unpublished). Choice of GAL4 drivers for the expression of human APC and/or other proteins was mainly determined by the severity of the phenotype.

### Example 4

RNA *in situ* was done essentially as described by Tautz and Pfeifle (1989) using FB10B cDNA clone of human APC as the probe. X-gal staining and immuno-histochemical staining were essentially as described (Ghysen and O'Kane, 1989; Patelet al., 1989). The lacZ reporter gene constructs used are UAS-lacZ (Brand and Perrimon, 1993) and *dpp-*lacZ (Blackman et al., 1991). The primary antibodies used are, anti-human APC (Hayashi et al., 1997), anti-Arm (Riggleman et al., 1990), anti-Dll (Vachon et al., 1992) and anti-Wg (Brook and Cohen, 1996). Anti-Arm and anti-Wg were obtained from the Development Studies Hybridoma Bank, University of Iowa, USA.

### Example 5

The adult appendages were processed for microscopy as described before (Shashidhara et al., 1999). For Scanning Electron microscopy (SEM), adult heads were air dried and coated with gold. SEM was carried out on Hitachi S520 machine.

### Example 6

To examine the effect of lowering endogenous β-catenin/Arm levels on hAPC induced phenotypes. *arm*^{*4*} allele of *arm* (http://flybase.bio.indiana.edu) was used along with hAPC/CBD, wherein flies carrying one copy of hAPC/CBD and one copy of *arm*^{*4*} allele allele were crossed to *ptc-* and *dpp*-GAL4 drivers. To rescue mutant phenotypes of *dAPC*, a III chromosome insertion of UAS-hAPC/FL transgene was combined with *dAPC*^{*Q8*} (Ahmed et al., 1998) and the resultant flies were crossed to different GAL4 strains, which were also heterozygous for *dAPC*^{*Q8*}. Homozygous flies were identified with the help of *ebony* marker associated with *dAPC*^{*Q8*}.

### Example 7

To screen for genetic modifiers of human APC function, ey-GAL4 was first crossed to a collection of *Drosophila* mutants (point mutations, deletions and P-lethal insertions) and then to UAS-hAPC/CBD. Following mutant stocks were used. *arm*^{*4*}, P-1783, P-1595, P-1659, P-0237, P-1568, Df(3L)AC1, Df(3L) 66C-G28, Df(3L) R-G7, Df(3L) 29A6, Df(3L) Cat, Df(3L) fz GF3b, Df(3L) fz M21, Df(3L) Ly, Df(3L) HR119, Df(3L) GN24, Df(3R) Scr, Df(3R) by 10, Df(3R) crb87-4, Df(3R) crb-87-5, Df(3R) by62, Df(3R) p-XT103, Df(3R) Cha7, Df(3R) red¹, Df(3R) C4, Df(3R) M-Kx1, Df(3R) B81, In(3LR) 270, In(3LR) 268 and In(3R) hb^{D1}. All the above-mentioned stocks are described in http://flybase.bio.indiana.edu. Alleles of *l(3)67E2* were generated in the laboratory (R Bajpai, unpublished).

### Example 8

For drug screening, drugs (at different concentrations) were added to fly food at <50° C and left over-night for drying. Care was taken to ensure homogenous mixing of the drug in the fly food. Blind-tests were carried out, wherein the vials with or without drugs were coded (with the help of other members of the lab). The crosses were always set-up in triplicates. The method of administering drugs described above is only an example and should not be limited to it as there can be other ways of administering drugs.

### Example 9

A method for determining the differential expression of genes following mis-expression of human APC in *Drosophila*. Briefly, RNA samples would be isolated from tissues expressing and not expressing human APC. They would be labeled by RT-PCR and resolved by gel electrophoresis or labeled probes would be hybridized to arrayed cDNA clones. This would allow us to identify genes that are differentially regulated by the expression of human APC. Such RNA-based approach of scanning the entire *Drosophila* genome would help identification of additional components of Wnt signal transduction pathway, which would provide suitable targets for drug screening against cancer and other conditions caused by over-activation of β-catenin.

### Example 10

A method for determining the modification and differential expression of proteins following mis-expression of human APC in *Drosophila*. Briefly, proteins would be isolated from tissues expressing or not expressing human APC and would be subjected to 2-D gel electrophoreses. The protein bands, which show differential mobility would be isolated and subjected to further analyses that includes MASS SPEC analyses and protein sequencing.

### List of Prior Art References:

1. Ahmed, Y., Hayashi, S., Levine, A. and Wieschaus, E. (1998) Regulation of armadillo by a *Drosophila* APC inhibits neuronal apoptosis during retinal development. *Cell* **93**, 1171-1182.
2. Bienz, M. (1999) APC: the plot thickens. *Curr Opin Genet Dev* **9,** 595-603.
3. Blackman, R. K., Sanicola, M., Raftery, L. A., Gillever, T. and Gelbart, W. M. (1991) An extensive 3' cis-regulatory region directs the imaginal disk expression of *decapentaplegic*, a member of the TGF-β family in *Drosophila*. *Development* **111**, 657-666.
4. Brand, A. H. and Perrimon, N. (1993) Targeted gene expression as a means of altering cell fates and generating dominant phenotypes. *Development* **118**, 401-415.
5. Brook, W. J. and Cohen, S. M. (1996) Antagonistic interactions between *wingless* and *decapentaplegic* responsible for dorsal-ventral pattern in the *Drosophila* leg. *Science* **273,** 1373-1377.
6. Campbell, G. and Tomlinson, A. (1998) The roles of the homeobox genes aristaless and Distal-less in patterning the legs and wings of *Drosophila*. *Development* **125**, 4483-4493.
7. Cooley, L., Kelley, R. and Spradling, A. (1988) Insertional mutagenesis of the *Drosophila* genome with single P elements. *Science* **239,** 1121-1128.
8. Cox, R.T., Kirkpatrick, C. and Peifer, M. (1996) Armadillo is required for adherens junction assembly, cell polarity, and morphogenesis during *Drosophila* embryogenesis. *J Cell Biol*. **134**, 133-48.
9. Diaz-Benjumea, F. J., Cohen, B. and Cohen, S. M. (1994) Cell interaction between compartments establishes the proximal-distal axis of *Drosophila* legs. *Nature* **372,** 175-179.
10. Fortini, M. E. and Bonini, N. M. (2000). Modeling human neurodegenerative diseases in *Drosophila*: on a wing and a prayer. *Trends Genet.* **16,** 161-167.
11. Frasch, M. (1995) Induction of visceral and cardiac mesoderm by ectodermal Dpp in the early *Drosophila* embryo. *Nature* **374**, 464-467.
12. Friedl, W., Meuschel, S., Caspari. R., Lambrati, C., Krieger, S., Sengteller, M. and Propping, P. (1996) Attenuated familial adenomatous polyposis due to a mutation in the 3 part of the APC gene. *Hum Genet* **97,** 579-584
13. Ghysen, A. and O'Kane, C. (1989) Neural enhancer-like elements as specific cell markers in *Drosophila*. *Development* **105**, 35-52.
14. Gorfinkiel, N., Morata, G. and Guerrero, I. (1997) The homeobox gene Distal-less induces ventral appendage development in *Drosophila*. *Genes Dev* **11**, 2259-2271.
15. Groden, J., Thliveris, A., Samowitz, W., Carlson, M., Gelbert, L., Albertsen, H., Joslyn, G., Stevens, J., Spirio, L. and Robertson, M. (1991) Identification and characterization of the familial adenomatous polyposis coli gene. *Cell* **66**, 589-600.
16. Gumbiner, B.M. (1995) Signal transduction of β-catenin. *Curr Opin Cell Biol*. **7**, 634-40.
17. Hayashi, S., Rubinfeld, B., Souza, B., Polakis, P., Wieschaus, E. and Levine, A. J. (1997) A *Drosophila* homolog of the tumor suppressor gene adenomatous polyposis coli down-regulates β-catenin but its zygotic expression is not essential for the regulation of Armadillo. *Proc Natl Acad Sci U S A* **94**, 242-247
18. Hazelett, D. J., Bourouis, M., Walldorf, U. and Treisman, J. E. (1998) *decapentaplegic* and *wingless* are regulated by *eyes absent* and *eyegone* and interact to direct the pattern of retinal differentiation in the eye disc. *Development* **125,** 785-789.
19. He T-C, Chan, T.A., Vogelstein, B. and Kinzler, K.W. (1999) PPARE is an APC-regulated target of nonsteroid anti-inflammatory drugs. *Cell* **99**, 335-345.
20. Held, L. I., Heup, M. A., Sappington, J. M. and Peters, S. (1994) Interaction of *decapentaplegic*, *wingless*, and *Distal-less* in the *Drosophila* leg. *Roux's Arch Dev Biol* **203**, 310-319.
21. Hyas, M., Tomhnson, I.P.M., Rowan, . Pignatelli, M and Bodmer. W.F. (2000) β-catenin mutations in cell lines established from human colorectal cancers. *Proc Natl Acad Sci U S A* **94**, 10330-10334.
22. Joslyn, G., Carlson, M., Thliveris, A., Albertsen, H., Gelbert, L., Samowitz. W., Groden, J., Stevens, J., Spirio, L. and Robertson, M. (1991) Identification of deletion mutations and three new genes at the familial polyposis locus. *Cell* **66**, 601-13.
23. Kinzler, K. W., Nilbert. M. C., Su. L. K., Vogelstein, B., Bryan, T. M., Levy, D. B., Smith, K. J., Preisinger, A. C., Hedge, P. and McKechnie, D. (1991) Identification of FAP locus genes from chromosome 5q21. *Science* **253**, 661-665.
24. Kopp, A., Blackman, R.K. and Duncan, I. (1999) Wingless, decapentaplegic and EGF receptor signaling pathways interact to specify dorso-ventral pattern in the adult abdomen of *Drosophila*. *Development* 1999 **126**, 3495-507.
25. Ma, C., Zhou, Y., Beachy, P.A. and Moses. K (1993) The segment polarity gene hedgehog is required for progression of the morphogenetic furrow in the developing *Drosophila* eye. *Cell* **75**, 927-938.
26. McCartney, B. M., Dierick, H. A., Kirkpatrick, C., Moline, M. M., Baas, A., Peifer, M. and Bejsovec, A. (1999) *Drosophila* APC2 is a cytoskeletally-associated protein that regulates Wingless signaling in the embryonic epidermis. *J Cell Biol* **146**, 1303-1318.
27. McCartney, B.M. and Peifer, M . (2000) Teaching tumour suppressors new tricks. *Nat Cell Biol* **2,** E58-E60
28. Miyoshi, Y., Nagase, H., Ando, H., Horii, A., Ichii, S., Nakatsuru, S., Aoki, T., Miki, Y., Mori, T. and Nakamura, Y. (1992) Somatic mutations of the APC gene in colorectal tumors: mutation cluster region in the APC gene. *Hum Mol Genet* **1,** 229-233.
29. Morimura, S., Maves, L., Chen, Y. and Hoffmann, F. M. (1996) *decapentaplegic* overexpression affects *Drosophila* wing and leg imaginal disc development and *wingless* expression. *Dev*. *Biol*. **177**, 136-151.
30. Nathke, I. S., Adams, C L., Polakis, P. Sellin, J. H. and Nelson. W. J. (1999) The adenomatous polyposis coli tumor suppressor protein localizes to plasma membrane sites involved in active cell migration. *J Neurosci.* **19,** 4229-4237.
31. Nellen, D., Affolter, M. and Basler, K. (1994) Direct and long-range action of a DPP morphogen gradient. *Cell* **78**, 225-237.
32. Neufeld, K and White, R.L. (1997) Nuclear and cytoplasmic localizations of the adenomatous polyposis coli protein. *Proc Natl Acad Sci U S A* **94**, 3034-3039.
33. Pai, L. M., Orsulic, S., Bejsovec, A. and Peifer, M. (1997) Negative regulation of Armadillo, a Wingless effector in *Drosophila*. *Development* **124**, 2255-2266.
34. Papkoff, J., Rubinfeld, B., Schryver, B. and Polakis, P. (1996) Wnt-1 regulates free pools of β-catenins and stabalizes APC-β-catenin complexes. *Mol*. *Cell. Biol*. **16,** 2128-2134.
35. Patel, N. H., Martin-Blanco, E., Coleman, K. G., Poole, S. J., Ellis, M. C., Kornberg, T. B. and Goodman, C. S. (1989) Expression of engrailed proteins in arthropods, annelids, and chordates. *Cell* **58**, 955-968.
36. Polakis, P. (1997) The adenomatous polyposis coli (APC) tumor suppressor. *Biochim Biophys Acta* **1332**, F127-F147.
37. Polakis, P. (1999) The oncogenic activation of β-catenin. *Curr Opin Genet Dev* **9**, 15-21.
38. Riggleman, B., Schedl, P. and Wieschaus, E. (1990) Spatial expression of the *Drosophila* segment polarity gene *armadillo* is posttranscriptionally regulated by Wingless. *Cell* **63,** 549-560
39. Rubinfeld, B., Albert, I., Porfiri, E., Fiol, C., Munemitsu, S. and Polakis, P. (1996) Stabilization of β-catenin by genetic defects in melanoma cell lines. *Science* **272,** 1023-1026.
40. Sanson, B., White, P. and Vincent, J. P. (1996) Uncoupling cadherin-based adhesion from Wingless signalling in *Drosophila*. *Nature* **383**, 627-630.
41. Scott, R.J., Froggatt, N.J., Trembath, R.C., Evans, D.J., Hodgson S.V. and Maher, E.R. (1996) Familial infiltrative fibromatosis (desmoid tumours) (MIM135290) caused by a recurrent 3 APC gene mutation. *Hum Mol Genet*. **5,** 1921-1924.
42. Shashidhara, L.S., Agrawal, N., Bajpai. R., Bharathi, V. and Sinha, P. (1999) Negative regulation of dorsoventral signaling by the homeotic gene *Ultrabithorax* during haltere development in *Drosophila. Dev. Biol*. **212**, 419-502.
43. Shih, I.M, Yu, J., He. T.C.. Vogelstein ,B. and Kinzler, K.W. (2000) The β-catenin binding domain of adenomatous polyposis coli is sufficient for tumor suppression. *Cancer Res* **60**, 1671-1676.
44. Shirras, A.D. and Couso, J.P. (1996) Cell fates in the adult abdomen of *Drosophila* are determined by wingless during pupal development. *Dev Biol*. **175**, 24-36.
45. Simmonds, A. J., Brook, W. J., Cohen, S. M. and Bell, J. B. (1995) Distinguishable functions for *engrailed* and *invected* in anterior-posterior patterning in the *Drosophila* wing. *Nature* **376**, 424-427.
46. Smits, R., Kielman, M. F., Breukel, C. Z., Zurcher, C., Neufeld, K., Jagmohan-Changur, S., Hofland, N., van Dijk, J., White, R., Edelmann, W., Kucherlapati, R., Khan, P. M. and Fodde, R. (1999) *Apc1638T*: a mouse model delineating critical domains of the adenomatous polyposis coli protein involved in tumorigenesis and development. *Genes Dev* **13,** 1309-1321
47. Steitz, M. C., Wickenheisser, J. K. and Siegfried, E. (1998) Overexpression of zeste white 3 blocks Wingless signaling in the *Drosophila* embryonic midgut. *Dev Biol* **197**, 218-233.
48. Tautz, D. and Pfeifle, C. (1989) A non-radioactive in situ hybridisation method for the localization of specific RNAs in *Drosophila* embryos reveals translational control of the segmentation gene hunchback. *Chromosoma* **98**, 81-85.
49. Treisman, J.E. and Rubin, G.M. (1995) *wingless* inhibits morphogenetic furrow movement in the *Drosophila* eye disc. *Development* **121**, 3519-3527.
50. Vachon, G.B., Cohen, B., Pfeifle. C., McGuffin, M.E., Botas, J. and Cohen, S. (1992) Homeotic genes of the bithorax complex repress lumb development in the abdomen of the *Drosophila* embryo through the target gene *Distal-less*. *Cell* **71,** 437-450.
51. Willert, K., Shibamoto, S. and Nusse, R. (1999) Wnt-induced dephosphorylation of Axin releases β-catenin from the axin complex. *Genes Dev* **13,** 1768-1773.
52. Yamaguchi, M., Hirose, F., Inoue, Y.H., Shiraki, M., Hayashi, Y., Nishi, Y. and Matsukage, A. (1999) Ectopic expression of human p53 inhibits entryinto S phase and induces apoptosis in the *Drosophila* eye imaginal disc. *Oncogene* 18, 6767-6775.
53. Yu, X., Waltzer, L. and Bienz. M. (1999) A new *Drosophila* APC homologue associated with adhesive zones of epithelial cells. *Nat Cell Biol 1,* 144-151.
54. zecca, m., basler, k. and struhl, g. (1996) direct and long-range action of a wingless morphogen gradient, *cell* 87, 833-844.

Organization Applicant
----------------------
      Street : Rafi Marg
      City : New Dehli 110001
      State :
      Country : India
      PostalCode : 110001
      PhoneNumber :
      FaxNumber :
      EmailAddress :
<110> OrganizationName : Council of scientific and industrial research

Application Project
-------------------
<120> Title : Invivo assay system for screening and validation of drugs abd o
ther substances
<130> AppFileReference : N644 Council of scientific
<140> CurrentAppNumber :
<141> CurrentFilingDate : ____-__-__

Earlier Applications
--------------------
<150> PriorAppNumber : 60/276,483
<151> PriorFilingDate : 2001-03-19

Sequence
--------
<213> OrganismName : APC protein
<400> PreSequenceString :
MAAASYDQLL KQVEALKMEN SNLRQELEDN SNHLTKLETE ASNMKEVLKQ LQGSIEDEAM      60
ASSGQIDLLE RLKELNLDSS NFPGVKLRSK MSLRSYGSRE GSVSSRSGEC SPVPMGSFPR     120
RGFVNGSRES TGYLEELEKE RSLLLADLDK EEKEKDWYYA QLQNLTKRID SLPLTENFSL     180
QTDMTRRQLE YEARQIRVAM EEQLGTCQDM EKRAQRRIAR IQQIEKDILR IRQLLQSQAT     240
EAERSSQNKH ETGSHDAERQ NEGQGVGEIN MATSGNGQGS TTRMDHETAS VLSSSSTHSA     300
PRRLTSHLGT KVEMVYSLLS MLGTHDKDDM SRTLLAMSSS QDSCISMRQS GCLPLLIQLL     360
HGNDKDSVLL GNSRGSKEAR ARASAALHNI IHSQPDDKRG RREIRVLHLL EQIRAYCETC     420
WEWQEAHEPG MDQDKNPMPA PVEHQICPAV CVLMKLSFDE EHRHAMNELG GLQAIAELLQ     480
VDCEMYGLTN DHYSITLRRY AGMALTNLTF GDVANKATLC SMKGCMRALV AQLKSESEDL     540
QQVIASVLRN LSWRADVNSK KTLREVGSVK ALMECALEVK KESTLKSVLS ALWNLSAHCT     600
ENKADICAVD GALAFLVGTL TYRSQTNTLA IIESGGGILR NVSSLIATNE DHRQILRENN     660
CLQTLLQHLK SHSLTIVSNA CGTLWNLSAR NPKDQEALWD MGAVSMLKNL IHSKHKMIAM     720
GSAAALRNLM ANRPAKYKDA NIMSPGSSLP SLHVRKQKAL EAELDAQHLS ETFDNIDNLS     780
PKASHRSKQR HKQSLYGDYV FDTNRHDDNR SDNFNTGNMT VLSPYLNTTV LPSSSSSRGS     840
LDSSRSEKDR SLERERGIGL GNYHPATENP GTSSKRGLQI STTAAQIAKV MEEVSAIHTS     900
QEDRSSGSTT ELHCVTDERN ALRRSSAAHT HSNTYNFTKS ENSNRTCSMP YAKLEYKRSS     960
NDSLNSVSSS DGYGKRGQMK PSIESYSEDD ESKFCSYGQY PADLAHKIHS ANHMDDNDGE    1020
LDTPINYSLK YSDEQLNSGR QSPSQNERWA RPKHIIEDEI KQSEQRQSRN QSTTYPVYTE    1080
STDDKHLKFQ PHFGQQECVS PYRSRGANGS ETNRVGSNHG INQNVSQSLC QEDDYEDDKP    1140
TNYSERYSEE EQHEEEERPT NYSIKYNEEK RHVDQPIDYS LKYATDIPSS QKQSFSFSKS    1200
SSGQSSKTEH MSSSSENTST PSSNAKRQNQ LHPSSAQSRS GQPQKAATCK VSSINQETIQ    1260
TYCVEDTPIC FSRCSSLSSL SSAEDEIGCN QTTQEADSAN TLQIAEIKEK IGTRSAEDPV    1320
SEVPAVSQHP RTKSSRLQGS SLSSESARHK AVEFSSGAKS PSKSGAQTPK SPPEHYVQET    1380
PLMFSRCTSV SSLDSFESRS IASSVQSEPC SGMVSGIISP SDLPDSPGQT MPPSRSKTPP    1440
PPPQTAQTKR EVPKNKAPTA EKRESGPKQA AVNAAVQRVQ VLPDADTLLH FATESTPDGF    1500
SCSSSLSALS LDEPFIQKDV ELRIMPPVQE NDNGNETESE QPKESNENQE KEAEKTIDSE    1560
KDLLDDSDDD DIEILEECII SAMPTKSSRK AKKPAQTASK LPPPVARKPS QLPVYKLLPS    1620
QNRLQPQKHV SFTPGDDMPR VYCVEGTPIN FSTATSLSDL TIESPPNELA AGEGVRGGAQ    1680
SGEFEKRDTI PTEGRSTDEA QGGKTSSVTI PELDDNKAEE GDILAECINS AMPKGKSHKP    1740
FRVKKIMDQV QQASASSSAP NKNQLDGKKK KPTSPVKPIP QNTEYRTRVR KNADSKNNLN    1800
AERVFSDNKD SKKQNLKNNS KDFNDKLPNN EDRVRGSFAF DSPHHYTPIE GTPYCFSRND    1860
SLSSLDFDDD DVDLSREKAE LRKAKENKES EAKVTSHTEL TSNQQSANKT QAIAKQPINR    1920
GQPKPILQKQ STFPQSSKDI PDRGAATDEK LQNFAIENTP VCFSHNSSLS SLSDIDQENN    1980
NKENEPIKET EPPDSQGEPS KPQASGYAPK SFHVEDTPVC FSRNSSLSSL SIDSEDDLLQ    2040
ECISSAMPKK KKPSRLKGDN EKHSPRNMGG ILGEDLTLDL KDIQRPDSEH GLSPDSENFD    2100

WKAIQEGANS IVSSLHQAAA AACLSRQASS DSDSILSLKS GISLGSPFHL TPDQEEKPFT 2160
SNKGPRILKP GEKSTLETKK IESESKGIKG GKKVYKSLIT GKVRSNSEIS GQMKQPLQAN 2220
MPSISRGRTM IHIPGVRNSS SSTSPVSKKG PPLKTPASKS PSEGQTATTS PRGAKPSVKS 2280
ELSPVARQTS QIGGSSKAPS RSGSRDSTPS RPAQQPLSRP IQSPGRNSIS PGRNGISPPN 2340
KLSQLPRTSS PSTASTKSSG SGKMSYTSPG RQMSQQNLTK QTGLSKNASS IPRSESASKG 2400
LNQMNNGNGA NKKVELSRMS STKSSGSESD RSERPVLVRQ STFIKEAPSP TLRRKLEESA 2460
SFESLSPSSR PASPTRSQAQ TPVLSPSLPD MSLSTHSSVQ AGGWRKLPPN LSPTIEYNDG 2520
RPAKRHDIAR SHSESPSRLP INRSGTWKRE HSKHSSSLPR VSTWRRTGSS SSILSASSES 2580
SEKAKSEDEK HVNSISGTKQ SKENQVSAKG TWRKIKENEF SPTNSTSQTV SSGATNGAES 2640
KTLIYQMAPA VSKTEDVWVR IEDCPINNPR SGRSPTGNTP PVIDSVSEKA NPNIKDSKDN 2700
QAKQNVGNGS VPMRTVGLEN RLNSFIQVDA PDQKGTEIKP GQNNPVPVSE TNESSIVERT 2760
PFSSSSSSKH SSPSGTVAAR VTPFNYNPSP RKSSADSTSA RPSQIPTPVN NNTKKRDSKT 2820
DSTESSGTQS PKRHSGSYLV TSV 2843
<212> Type : PRT
<211> Length : 2843
SequenceName : SEQ ID N°1 full-length sequence of APCProtein (Kinzler e
t al. 1991)
SequenceDescription :

Organization Applicant
-----------------------
      Street : Rafi Marg
      City : New Dehli
      State :
      Country : India
      PostalCode : 110 001
      PhoneNumber :
      FaxNumber :
      EmailAddress :
<110> OrganizationName : Council of scientific and industrial research

Application Project
-------------------
<120> Title : in vivo assay system for screening and validation of drugs and
other
substances
<130> AppFileReference : N 644
<140> CurrentAppNumber :
<141> CurrentFilingDate : ____-__-__

Sequence
--------
<213> OrganismName : APC Protein from amino-acids 959 to 1870
<400> PreSequenceString :
SSNDSLNSVS SSDGYGKRGQ MKPSIESYSE DDESKFCSYG QYPADLAHKI HSANHMDDND      60
GELDTPINYS LKYSDEQLNS GRQSPSQNER WARPKHIIED EIKQSEQRQS RNQSTTYPVY     120
TESTDDKHLK FQPHFGQQEC VSPYRSRGAN GSETNRVGSN HGINQNVSQS LCQEDDYEDD     180
KPTNYSERYS EEEQHEEEER PTNYSIKYNE EKRHVDQPID YSLKYATDIP SSQKQSFSFS     240
KSSSGQSSKT EHMSSSSENT STPSSNAKRQ NQLHPSSAQS RSGQPQKAAT CKVSSINQET     300
IQTYCVEDTP ICFSRCSSLS SLSSAEDEIG CNQTTQEADS ANTLQIAEIK EKIGTRSAED     360
PVSEVPAVSQ HPRTKSSRLQ GSSLSSESAR HKAVEFSSGA KSPSKSGAQT PKSPPEHYVQ     420
ETPLMFSRCT SVSSLDSFES RSIASSVQSE PCSGMVSGII SPSDLPDSPG QTMPPSRSKT     480
PPPPPQTAQT KREVPKNKAP TAEKRESGPK QAAVNAAVQR VQVLPDADTL LHFATESTPD     540
GFSCSSSLSA LSLDEPFIQK DVELRIMPPV QENDNGNETE SEQPKESNEN QEKEAEKTID     600
SEKDLLDDSD DDDIEILEEC IISAMPTKSS RKAKKPAQTA SKLPPPVARK PSQLPVYKLL     660
PSQNRLQPQK HVSFTPGDDM PRVYCVEGTP INFSTATSLS DLTIESPPNE LAAGEGVRGG     720
AQSGEFEKRD TIPTEGRSTD EAQGGKTSSV TIPELDDNKA EEGDILAECI NSAMPKGKSH     780
KPFRVKKIMD QVQQASASSS APNKNQLDGK KKKPTSPVKP IPQNTEYRTR VRKNADSKNN     840
LNAERVFSDN KDSKKQNLKN NSKDFNDKLP NNEDRVRGSF AFDSPHHYTP IEGTPYCFSR     900
NDSLSSLDFD DD                                                         912
<212> Type : PRT
<211> Length : 912
      SequenceName : SEQ ID N°2 sequence of APC protein (Kinzler et al. 1991)
 from
amino-acids 959 to 1870
      SequenceDescription :

Organization Applicant
----------------------
      Street : Rafi Marg
      City : New Dehli
      State :
      Country : India
      PostalCode : 110 001
      PhoneNumber :
      FaxNumber :
      EmailAddress :
<110> OrganizationName : Council of scientific and industrial research

Application Project
-------------------
<120> Title : In vivo assay system for screening and bvalidation of drugs and
 other
substances
<130> AppFileReference : N 644
<140> CurrentAppNumber :
<141> CurrentFilingDate : ____-__-__

Sequence
--------
<213> OrganismName : APC protein from amino-acids 1 to 767
<400> PreSequenceString :
MAAASYDQLL KQVEALKMEN SNLRQELEDN SNHLTKLETE ASNMKEVLKQ LQGSIEDEAM      60
ASSGQIDLLE RLKELNLDSS NFPGVKLRSK MSLRSYGSRE GSVSSRSGEC SPVPMGSFPR     120
RGFVNGSRES TGYLEELEKE RSLLLADLDK EEKEKDWYYA QLQNLTKRID SLPLTENFSL     180
QTDMTRRQLE YEARQIRVAM EEQLGTCQDM EKRAQRRIAR IQQIEKDILR IRQLLQSQAT     240
EAERSSQNKH ETGSHDAERQ NEGQGVGEIN MATSGNGQGS TTRMDHETAS VLSSSSTHSA     300
PRRLTSHLGT KVEMVYSLLS MLGTHDKDDM SRTLLAMSSS QDSCISMRQS GCLPLLIQLL     360
HGNDKDSVLL GNSRGSKEAR ARASAALHNI IHSQPDDKRG RREIRVLHLL EQIRAYCETC     420
WEWQEAHEPG MDQDKNPMPA PVEHQICPAV CVLMKLSFDE EHRHAMNELG GLQAIAELLQ     480
VDCEMYGLTN DHYSITLRRY AGMALTNLTF GDVANKATLC SMKGCMRALV AQLKSESEDL     540
QQVIASVLRN LSWRADVNSK KTLREVGSVK ALMECALEVK KESTLKSVLS ALWNLSAHCT     600
ENKADICAVD GALAFLVGTL TYRSQTNTLA IIESGGGILR NVSSLIATNE DHRQILRENN     660
CLQTLLQHLK SHSLTIVSNA CGTLWNLSAR NPKDQEALWD MGAVSMLKNL IHSKHKMIAM     720
GSAAALRNLM ANRPAKYKDA NIMSPGSSLP SLHVRKQKAL EAELDAQ                  767
<212> Type : PRT
<211> Length : 767
      SequenceName : SEQ ID N°3 of APC protein (Kinzler et al. 1991) from ami
no-acids 1to
767
      SequenceDescription :

SEQUENCE COMPARISON 1

CLUSTAL W (1.81) multiple sequence alignment of β-catenin sequences of human, mouse, rat, Drosophila, Hydra and C. elegans.

gib|399310_MOUSE ---------MATQADLME-----LDMAMEPDRKAAVSHWQQQSYL-DSGIH 36
gic|9972860_RAT ---------MATQADLME-----LDMAMEPDRKAAVSHWQQQSYL-DSGIH 36
gia|461854_HUMAN ---------MATQADLME-----LDMAMEPDRKAAVSHWQQQSYL-DSGIH 36
gid|479677 DROSO ---------MATQADLME-----LDMAMEPDRKAAVSHWQQQSYL-DSGIH 36
gie|399056_ARM_MUSDO ---------MSHNNQYNP-----PDLPPMVSAKEQTLMWQQNSYLVDSGIH 37
gii|1407601_Hydra MMEDSTAQMRYQYQHQQSYDQGRDVRMLEDRRNKLMMMLESNQPNRQQIQ 50
gif|7505312_T23341 ----------------------------------MLLHSTNSYS------ 10
gig|3153823_BAR-1Celegans ----------------------MDLDPNLVINHDDTNLSEASFTMEQHTS 28
gih|2343265_wrm-1 ----------------------MDVDCAETFSQPCTPLNFNPMTPSTSRV 28

gib|399310_MOUSE SGATTTAPSLSGKGNPEEEDVDT-------SQVL------------YEWE 67
gic|9972860_RAT SGATTTAPSLSGKGNPEEEDVDT-------SQVL------------YEWE 67
gia|461854_HUMAN SGATTTAPSLSGKGNPEEEDVDT-------SQVL------------YEWE 67
gid|479677 DROSO SGATTTAPSLSGKGNPEDEDVDT-------NQVL------------YEWE 67
gie|399056_ARM_MUSDO SGAVTQAPSLSGK---EDEEMEG-------DPLM------------FDLD 65
gii|1407601_Hydra MEIRSVETQLQQMRMQKQGMVDFNMHHQQMHPAMNKMNQTAVWNQGYNID 100
gif|7505312_T23341 --------------------------------------------------
gig|3153823_BAR-1Celegans SYSDIHMGSTPCTGHRKVDMWRN--------------------------- 51
gih|2343265_wrm-1 STPVRPSSTMSARQYSGSPFKAQP-------------------------- 52

gib|399310_MOUSE QGFS------QSFTQEQVA--------------------DIDGQYAMTRA 91
gic|9972860_RAT QGFS------QSFTQEQVA--------------------DIDGQYAMTRA 91
gia|461854_HUMAN QGFS------QSFTQEQVA--------------------DIDGQYAMTRA 91
gid|479677 DROSO QGFS------QSFTQDQVA--------------------DIDGQYAMTRA 91
gie|399056_ARM_MUSDO TGFP------QNFTQDQVD--------------------DMNQQLSQTRS 89
gii|1407601_Hydra SGIQTAAPTVKGYDDDEVASHHSYQQIEWDQFSGEPMDTAINDQFNNNRS 150
gif|7505312_T23341 -----------IFTDHEVE-------------------------------TRT 21
gig|3153823_BAR-1Celegans ----------HNFDSGFQT-------------------------------M 61
gih|2343265_wrm-1 ----------QNMEPSNSR-------------------------------V 62

gib|399310_MOUSE QRVRAAMFPETLDEGMQIPSTQFDAAHPTNVQRLAEPSQMLKHAVVNLIN 141
gic|9972860_RAT QRVRAAMFPETLDEGMQIPSTQFDAAHPTNVQRLAEPSQMLKHAVVNLIN 141
gia|461854_HUMAN QRVRAAMFPETLDEGMQIPSTQFDAAHPTNVQRLAEPSQMLKHAVVNLIN 141
gid|479677 DROSO QRVRAAMFPETLDEGMQIPSTQFDSAHPTNVQRLAEPSQMLKHAVVNLIN 141
gie|399056_ARM_MUSDO QRVRAAMFPETLEEGIEIPSTQFDPQQPTAVQRLAEPSQMLKHAVVNLIN 139
gii|1407601_Hydra QRARTGMFPEAMHENMELSHAQIHNNNSAVPQRLAEPTQMLKNNVIDLIN 200
gif|7505312_T23341 SRIRSAMFPDWIPP---TSAAEATNSTTSIVEMMQMPTQQLKQSVMDLLT 68
gig|3153823_BAR-1Celegans NHSEAPSIISSLHPSSHLSGMSSMADYEPIPTLSDQQKQKFDGITQNQAD 111
gih|2343265_wrm-1 QELREAAVGKRSYTNAWMQGTYAPPQMAGQQQRFSRPPSVIGSTMSHMTN 112

gib|399310_MOUSE YQDDAELATRAIPELTKLLNDEDQVVVNKAAVMVHQLSKKEASRHAIMRS 191
gic|9972860_RAT YQDDAELATRAIPELTKLLNDEDQVVVNKAAVMVHQLSKKEASRHAIMRS 191
gia|461854_HUMAN YQDDAELATRAIPELTKLLNDEDQVVVNKAAVMVHQLSKKEASRHAIMRS 191
gid|479677 DROSO YQDDAELATRAIPELTKLLNDEDQVVVNKAAVMVHQLSKKEASRHAIMRS 191
gie|399056_ARM_MUSDO YQDDAELATRAIPELIKLLNDEDQVVVSQAAMMVHQLSKKEASRHAITNS 189
gii|1407601_Hydra YQDETDVALPAVPELARLLCNSDAQTIHQASIMVNQLTKKEASCYAVMNN 250
gif|7505312_T23341 YEGSNDMSGLSLPDLVKLMCDHDESVVARAVHRAYMLSREDPNFFNAPGF 118
gig|3153823_BAR-1Celegans GQYN---TVRAIPELTMLMKDQDNEVVHKAVILMQNIAKMECDPMRRQNE 158
gih|2343265_wrm-1 MSEMTAYSYGGLSMLSVNTEMGEFNNFVNQAPYQPALTRVSQVSENQDPT 162

gib|399310_MOUSE PQ-MVSAIVRTMQ-----NTNDVETARCTAGTLHNLSHHREGLLAIFKSG 235
gic|9972860_RAT PQ-MVSAIVRTMQ-----NTNDVETARCTAGTLHNLSHHREGLLAIFKSG 235
gla|461854_HUMAN PQ-MVSAIVRTMQ-----NTNDVETARCTAGTLHNLSHHREGLLAIFKSG 235
gid|479677 DROSO PQ-MVSAIVRTMQ-----NTNDVETARCTAGTLHNLSHHREGLLAIFKSG 235
gie|399056_ARM_MUSDO PQ-MVAALVRAIS-----NSNDLESTKAAVGTLHNLSHHRQGLLAIFKSG 233
gii|1407601_Hydra TN-IVAALVGVTA-----TSNDGETIRNVVGALHNMSHHRQGLMAIFKCS 294
gif|7505312_T23341 DHRSFVEALMAAS-----KSSNVNVRPNAIGALSHMSEQRGGPLLIFRSG 163
gig|3153823_BAR-1Celegans AFIVDPRVIFTLRDLLRDKVEFPNIIRCTLGTFFHICNRQEGIDLVTRAI 208
gih|2343265_wrm-1 NRQYPMTAPEIIEN---LESTELINQAAAIRALEPIVKAG-GMLQTWGPK 208

gib|399310_MOUSE G-----IPALVKMLGSPVDS--VLFYAITTLHNLLLHQE---GAKMAVRL 275
gic|9972860_RAT G-----IPALVKMLGSPVDS--VLFYAITTLHNLLLHQE---GAKMAVRL 275
gla|461854_HUMAN G-----IPALVKMLGSPVDS--VLFYAITTLHNLLLHQE---GAKMAVRL 275
gid|479677 DROSO G-----IPALVKMLGSPVDS--VLFYAITTLHNLLLHQE---GAKMAVRL 275
gie|399056_ARM_MUSDO G-----IPALVKLLSSPVES--VLFYAITTLHNLLLHQD---GSKMAVRL 273
gii|1407601_Hydra G-----IPALVKLLGHRIEA--VVFYAITTLHNLLLHQE---GAKMAVRL 334
gif|7505312_T23341 G-----LAEIIRMLYDSLES--VVHYAVTTLRNLLMHVS---DSRAQARA 203
gig|3153823_BAR-1Celegans AEQPDIIPNLIRHIGTYPSS--IYKYAILTMHSILSDKQRGGQSVIIARQ 256
gih|2343265_wrm-1 GAEPIIRALFQVLIPRPVENENVIRKAFEILHHSILLSN-----KEIRRI 253

gib|399310_MOUSE AGGLQKMVALLNKTN-VKFLAITTDCLQILAYGNQESKLIIILASGGPQAL 324
gic|9972860_RAT AGGLQKMVALLNKTN-VKFLAITTDCLQILAYGNQESKLIIILASGGPQAL 324
gla|461854_HUMAN AGGLQKMVALLNKTN-VKFLAITTDCLQILAYGNQESKLIIILASGGPQAL 324
gid|479677 DROSO AGGLQKMVALLNKTN-VKFLAITTDCLQILAYGNQESKLIIILASGGPQAL 324
gie|399056_ARM_MUSDO AGGLQKMVTLLQRNN-VKFLAIVTDCLQILAYGNQESKLIIILASGGPNEL 322
gii|1407601_Hydra ALGLQKMVSLLQRPK-VKFLAIVTDCLQILAYGNQESKLIIILSSGGPAEL 383
gif|7505312_T23341 LNAVEALTPHLHKTN-PKLLAQVADGLYFLLIDDAPSKITFLSLLGPQIL 252
gig|3153823_BAR-1Celegans QDAITHVTPWLEAEKSEKLLPVIVDLIRVLCEKNTEQKIKFVKMGGPQKL 306
gih|2343265_wrm-1 DRMFFRLNAALMDPNGPPVFNVPKPYSIYEIVMTRAIQLDTKFESSAMVL 303

gib|399310_MOUSE VNIMRTY-TYEKLLWTTSRVLKVLSVCSSNKPAIVEAGGMQALGLHLTDP 373
gic|9972860_RAT VNIMRTY-TYEKLLWTTSRVLKVLSVCSSNKPAIVEAGGMQALGPHLTDP 373
gla|461854_HUMAN VNIMRTY-TYEKLLWTTSRVLKVLSVCSSNKPAIVEAGGMQALGLHLTDP 373
gid|479677 DROSO VNIMRTY-SYEKLLWTTSRVLKVLSVCSSNKPAIVEAGGMQALGLHLTDS 373
gie|399056_ARM_MUSDO VRIMRSY-DYEKLLWTTSRVLKVLSVCSSNKPAIVDAGGMQALAMHLSNP 371
gii|1407601_Hydra VRIMRSY-TYEKLLYTTCRVLKVLSVCSSNKPAIVEAGGMQALAHYLSHQ 432
gif|7505312_T23341 VSILREYSDHRKLIYTVVRCIRSLSVCPSNKPALISLGCLPALYVELCTA 302
gig|3153823_BAR-1Celegans LMLLQHR-VYENLLWRTTQLLKTFSNFD--APNLVAFGGRQILANLLSHG 353
gih|2343265_wrm-1 LVHLCCKPHFMKIFFGEDETQQSPAHRRLHKIVIEFAIG--NLRRPETKS 351

gib|399310_MOUSE -SQRLVQNCLWTLRNLSDAATKQEGMEGLLGTLVQLLGSDDINVVTCAAG 422
gic|9972860_RAT -SQRLVQNCLWTLRNLSDAATKQEGMEGLLGTLVQLLGSDDINVVTCAAG 422
gla|461854_HUMAN -SQRLVQNCLWTLRNLSDAATKQEGMEGLLGTLVQLLGSDDINVVTCAAG 422
gid|479677 DROSO -SQRLVQNCLWTLRNLSDAATKQEGMEGLLGTLVQLLGSDDINVVTCAAG 422
gie|399056_ARM_MUSDO -SPRLVQNCLWTLRNLSDAATKVDGLEPLLQSLVQVLASTDVNVVTCAAG 420
gii|1407601_Hydra -STRLVQNCLWTLRNLSDVATKQDGLEGLLQMLVQLLSSNDINVVTCVSG 481
gif|7505312_T23341 KDERSQTAILVAMRNLSDSATNEENLTQLIIKLLEIIRVANDGMTACACG 352
gig|3153823_BAR-1Celegans -SPRLVQSTLETLRNISDVPSKIK-EDLLLKSLLELVNSRNTTIRLYSAQ 401
gih|2343265_wrm-1 FNKGLCVSIIKNLSNKNATIKDMSEFLGVVSLFHQIMQNEVIHEDLLWST 401

gib|399310_MOUSE ILSN-LTCNNYKNKMMVCQVGGIEALVRTVLRAG------------DRED 459
gic|9972860_RAT ILSN-LTCNNYKNKMMVCQVGGIEALVRTVLRAG------------DRED 459
gla|461854_HUMAN ILSN-LTCNNYKNKMMVCQVGGIEALVRTVLRAG------------DRED 459
gid|479677 DROSO ILSN-LTCNNYKNKMMVCQVGGIEALVRTVLRAG------------DRED 459
gie|399056_ARM_MUSDO ILSN-LTCNNQRNKATVCQVGGVDALVRTIINAG------------DREE 457
gii|1407601_Hydra ILSN-LTCNNPRNKQVVFQVGGIEALVRTIINAG------------DREE 518
gif|7505312_T23341 TLSN-LTCNNTRNKQTVCSHGGIDALVTAIRRLP------------EVEE 389
gig|3153823_BAR-1Celegans IMSN-LVANNNHNKEFMCGNNGVILLVRLLTIATKEMGDLSDKEAQQMED 450
gih|2343265_wrm-1 [?]QALTVFTGDVYNGTHFVQMGGRQVLS[?]L[?]HGST-LLHELLTTLRRVGD 451

gib|399310_MOUSE ITEPAICALRHLTSRHQEAEMAQNAVR--LHYGLPVVVKLLHPPSHWPLI 507
gic|9972860_RAT ITEPAICALRHLTSRHQEAEMAQNAVR--LHYGLPVVVKLLHPPSHWPLI 507
gla|461854_HUMAN ITEPAICALRHLTSRHQEAEMAQNAVR--LHYGLPVVVKLLHPPSHWPLI 507
gid|479677 DROSO ITEPAICALRHLTSRHQEAEMAQNAVR--LHYGLPVVVKLLHPPSHWPLI 507
gie|399056_ARM_MUSDO ITEPAVCALRHLTTRHADSEMAQNAVR--LNYGLSVIVKLLHPPSRWPLI 505
gii|1407601_Hydra ITEPAVCALRHLTSRHPDAEHAENGVR--LHYGIPILVKLLNPPSRWPLI 566
gif|7505312_T23341 VTEPALCALRHCTARHSLAEEAQSELR--FCQAFPVILDQLET-LRTPVI 436
gig|3153823_BAR-1Celegans YIESLICTLRHLCVGHPMSDKVQAFVFRDPALFLHKLLTMRPVLLKHTLS 500
gih|2343265_wrm-1 LPAIQEQDMKESIHCIVQLIGCSDVTIVELATGTLRNIGLHNKMNKAFMV 501

gib|399310_MOUSE KATVGLIRNLALCPANHAPLREQGAIPRLVQLLVRAHQDTQR-RTSMG-- 554
gic|9972860_RAT KATVGLIRNLALCPANHAPLREQGAIPRLVQLLVRAHQDTQR-RTSMG-- 554
gla|461854_HUMAN KATVGLIRNLALCPANHAPLREQGAIPRLVQLLVRAHQDTQR-RTSMG-- 554
gid|479677 DROSO KATVGLIRNLALCPANHAPLREQGAIPRLVQLLVRAHQDTQR-RTSIG-- 554
gie|399056_ARM_MUSDO KAAIGLVRNLALCPANAAPLREHGAIHHLVRLLMRAFQDTERQRSSVATT 555
gii|1407601_Hydra KAVVGLIRNLGLCPSNHTPIRDQGGLPKLVQLLMKSYQDIQR----RGP- 611
gif|7505312_T23341 KAALGVIRNSALLQTNLIELTQEQTAN--GHTAVSLTMDILR-RAITAIE 483
gig|3153823_BAR-1Celegans LLLKVVSQHALLAPFRSCRIGDKGFVEQLIHILRVACTQLNVQESIEGVR 550
gih|2343265_wrm-1 QDGVTSHAIAVLRTSEQFTYQPHANIDLYRKQILSIYENCLSVLNNVTSM 551

gib|399310_MOUSE GTQQ---QFVEGVRMEEI-VEGCTGALHILARDVHNRIVIRGLN------ 594
gic|9972860_RAT GTQQ---QFVEGVRMEEI-VEGCTGALHILARDVHNRIVIRGLN------ 594
gla|461854_HUMAN GTQQ---QFVEGVRMEEI-VEGCTGALHILARDVHNRIVIRGLN------ 594
gid|479677 DROSO GTQQ---QFVEGVRMEEI-VEGCTGALHILARDIHNRIVIRGLN------ 594
gie|399056_ARM_MUSDO GSQQP-AAYADGVRMEEI-VEGTVGALHILARESHNRALIRQQS------ 597
gii|1407601_Hydra GAQN----MQDGVRMEEI-VEGTVGALHILAREALNRSIIRDLN------ 650
gif|7505312_T23341 ENPD---IAVDGVPMWGV-IEGAVSALHQLANHPAVAAACCDDIGQVGNP 529
gig|3153823_BAR-1Celegans KDII-HLCIQILRWITR-DQDILNEVVFFLQTPENSRMGDGHT------ 592
gih|2343265_wrm-1 APQDIKESAVSACRMISENADSAYVLLHYFNVGNRKCRKLAVTVMKRVIE 601

gib|399310_MOUSE TIPLFVQLLYSPIEN----------IQRVAAGVLCELAQDKEAAEAIEAE 634
gic|9972860_RAT TIPLFVQLLYSPIEN----------IQRVAAGVLCELAQDKEAAEAIEAE 634
gla|461854_HUMAN TIPLFVQLLYSPIEN----------IQRVAAGVLCELAQDKEAAEAIEAE 634
gid|479677 DROSO TIPLFVQLLYSPIEN----------IQRVAAGVLCDVAQDKEAAEAIEAE 634
gie|399056_ARM_MUSDO VIPIFVRLLFNEIEN----------IQRVAAGVLCELAADKEGAEIIEQE 637
gii|1407601_Hydra CIPTFVQLLYSEVEN----------IVRVAAGVLCELAQDKEGADAIERE 690
gif|7505312_T23341 ECPPFLDLLHRLLAHPRLGSMDDEVLEREILGLLYQLSKRPDGARAVEST 579
gig|3153823_BAR-1Celegans -LPIFVLQKANVEEN----------TKSSALALIYNLMHHEQMANVLDRD 631
gih|2343265_wrm-1 TVPAFADPFVDLLGTTN----EPLPILLLQRAFQSLDEWRKTSVEMMNCD 647

gib|399310_MOUSE GATAPLTE---LLHSRNEGVATYAAAVLFRMSEDKPQD------YKKRLS 675
gic|9972860_RAT GATAPLTE---LLHSRNEGVATYAAAVLFRMSEDKPQD------YKKRLS 675
gla|461854_HUMAN GATAPLTE---LLHSRNEGVATYAAAVLFRMSEDKPQD------YKKRLS 675
gid|479677 DROSO GATAPLTE---LLHSRNEGVATYAAAVLFRMSEDKPQD------YKKRLS 675
gie|399056_ARM_MUSDO GATGPLTD---LLHSRNEGVATYAAAVLFRMSEDKPQD------YKKRLS 678
gii|1407601_Hydra GATTILTE---LLHSRNDGIAAYARAVLFRMSEDKSQD------YKKRLS 731
gif|7505312_T23341 GVSALLME---SRGSQYKSVVTYANGVLSNLKRGDSAA------IMN--- 617
gig|3153823_BAR-1Celegans DVLVKMLQNVQMQSQTHPELASLANNILKMMYEKREKTRNTLPRYNSYLE 681
gih|2343265_wrm-1 GRSAEQRRELDDRRKDHEDIVKRSVGLLTNLCSQANPR------FFHSLK 691

gib|399310_MOUSE VELTS-SLFRTEPMAWNETADLGLDIGAQ------GEALGYR----QDDP 714
gic|9972860_RAT VELTS-SLFRTEPMAWNETADLGLDIGAQ------GEALGYR----QDDP 714
gla|461854_HUMAN VELTS-SLFRTEPMAWNETADLGLDIGAQ------GEPLGYR----QDDP 714
gid|479677 DROSO VELTS-SLFRTEPMPWNEAADLGLDIGAQ------GEPLGYR----QDDS 714
gie|399056_ARM_MUSDO IELTTNSLLREDNNIWGN-ADLGLGPDLQDML---GPEQAYEGLYGQGPP 724
gii|1407601_Hydra VELTS-SLFR-DDVPWEPGNTEMADILTS---------QSYA-------D 763
gif|7505312_T23341 --MSN----SYDYEMSGSAADWQRDGLEP-----------------ELFA 644
gig|3153823_BAR-1Celegans SQFGHMGMTTPPSEALNSSGEVCEGAGEQWSTPLTDDTMMDSYCNSSGPD 731
gih|2343265_wrm-1 LVLTNGTLNPFQWLTHEMSDGILQEWLAFILS------------ICSPDE 729

gib|399310_MOUSE SYRSFHSG---GYGQDALGMDPMMEHEMG---GHHPGADYPVDGLP---- 754

gic|9972860_RAT SYRSFHSG---GYGQDALGMDPMMEHEMG---GHHPGADYPVDGLP---- 754
gla|461854_HUMAN SYRSFHSG---GYGQDALGMDPMMEHEMG---GHHPGADYPVDGLP---- 754
gid|479677 DROSO SYRSFHAA---GYGQDAMGMDSMMDHDMG---GHHPGADYPVDGLP---- 754
gie|399056_ARM_MUSDO SVHSSHGGRAFQQGYDTLPIDSMQGLEISSPVGGGGGGGAPPSAAPTSPY 774
gii|1407601_Hydra EIYSPHVS------QNNLSYNPNSYQHQQ------------SGMFP---- 791
gif|7505312_T23341 EMYPTNDG----------GHSESINMALN--------------------- 663
gig|3153823_BAR-1Celegans SSKPYNSP--MYHSPPAMYPEYSIGPPETYLDPHATASCYPRPTPPQYN- 778
gih|2343265_wrm-1 SLQTFMMYR--FLEQAKMTEAFFAELKARRQNSNIQTMLSKIIDLG---- 773

gib|399310_MOUSE ------------DLGHAQDLMDGLPPGDSNQLAWFDTDL--- 781
gic|9972860_RAT ------------DLGHAQDLMDGLPPGDSNQLAWFDTDL--- 781
gla|461854_HUMAN ------------DLGHAQDLMDGLPPGDSNQLAWFDTDL--- 781
gid|479677 DROSO ------------DLSHAQDLMDGLPPGDSNQLAWFDTDL--- 781
gie|399056_ARM_MUSDO SMDMDVGEIDAGALNFDLDAMPTPPNDNNNLAAWYDTDC--- 813
gii|1407601_Hydra ------------------------QMQNNVTQGWFDPDL--- 806
gif|7505312_T23341 ------------------------NSQMRPNHNWYDTDL--- 678
gig|3153823_BAR-1Celegans ---------SYDRSPPVYNDLPSNPGPSSHSSDYYPSRNSRF 811
gih|2343265_wrm-1 ----------------RHQQRIVSQQHQQHQMQHHRQLM--- 796

### Annex to the application documents-subsequently filed sequences kisting

SEQUENCE LISTING

<110>  Council of scientific and industrial research

<120>  Invivo assay system for screening and validation of drugs abd other su
bstances

<130>  N644 Council of scientific

<140>  EP 02290571.5
<141>  2002-03-07

<150>  60/276,483
<151>  2001-03-19

<160>  3

<170>  PatentIn version 3.1

<210>  1
<211>  2843
<212>  PRT
<213>  APC protein

<400>  1

Met Ala Ala Ala Ser Tyr Asp Gln Leu Leu Lys Gln Val Glu Ala Leu
1               5                   10                  15

Lys Met Glu Asn Ser Asn Leu Arg Gln Glu Leu Glu Asp Asn Ser Asn
            20                  25                  30

His Leu Thr Lys Leu Glu Thr Glu Ala Ser Asn Met Lys Glu Val Leu
        35                  40                  45

Lys Gln Leu Gln Gly Ser Ile Glu Asp Glu Ala Met Ala Ser Ser Gly
    50                  55                  60

Gln Ile Asp Leu Leu Glu Arg Leu Lys Glu Leu Asn Leu Asp Ser Ser
65                  70                  75                  80

Asn Phe Pro Gly Val Lys Leu Arg Ser Lys Met Ser Leu Arg Ser Tyr
                85                  90                  95

Gly Ser Arg Glu Gly Ser Val Ser Ser Arg Ser Gly Glu Cys Ser Pro
            100                 105                 110

Val Pro Met Gly Ser Phe Pro Arg Arg Gly Phe Val Asn Gly Ser Arg
        115                 120                 125

Glu Ser Thr Gly Tyr Leu Glu Glu Leu Glu Lys Glu Arg Ser Leu Leu
    130                 135                 140

Leu Ala Asp Leu Asp Lys Glu Glu Lys Glu Lys Asp Trp Tyr Tyr Ala
145                 150                 155                 160

Gln Leu Gln Asn Leu Thr Lys Arg Ile Asp Ser Leu Pro Leu Thr Glu
                165                 170                 175

Asn Phe Ser Leu Gln Thr Asp Met Thr Arg Arg Gln Leu Glu Tyr Glu
            180                 185                 190

Ala Arg Gln Ile Arg Val Ala Met Glu Glu Gln Leu Gly Thr Cys Gln
        195                 200                 205

Asp Met Glu Lys Arg Ala Gln Arg Arg Ile Ala Arg Ile Gln Gln Ile
    210                 215                 220

Glu Lys Asp Ile Leu Arg Ile Arg Gln Leu Leu Gln Ser Gln Ala Thr
225                 230                 235                 240

Glu Ala Glu Arg Ser Ser Gln Asn Lys His Glu Thr Gly Ser His Asp
                245                 250                 255

Ala Glu Arg Gln Asn Glu Gly Gln Gly Val Gly Glu Ile Asn Met Ala
            260                 265                 270

Thr Ser Gly Asn Gly Gln Gly Ser Thr Thr Arg Met Asp His Glu Thr
        275                 280                 285

Ala Ser Val Leu Ser Ser Ser Ser Thr His Ser Ala Pro Arg Arg Leu
    290                 295                 300

Thr Ser His Leu Gly Thr Lys Val Glu Met Val Tyr Ser Leu Leu Ser
305                 310                 315                 320

Met Leu Gly Thr His Asp Lys Asp Asp Met Ser Arg Thr Leu Leu Ala
                325                 330                 335

Met Ser Ser Ser Gln Asp Ser Cys Ile Ser Met Arg Gln Ser Gly Cys
            340                 345                 350

Leu Pro Leu Leu Ile Gln Leu Leu His Gly Asn Asp Lys Asp Ser Val
        355                 360                 365

Leu Leu Gly Asn Ser Arg Gly Ser Lys Glu Ala Arg Ala Arg Ala Ser
    370                 375                 380

Ala Ala Leu His Asn Ile Ile His Ser Gln Pro Asp Asp Lys Arg Gly
385                 390                 395                 400

Arg Arg Glu Ile Arg Val Leu His Leu Leu Glu Gln Ile Arg Ala Tyr
                405                 410                 415

Cys Glu Thr Cys Trp Glu Trp Gln Glu Ala His Glu Pro Gly Met Asp
            420                 425                 430

Gln Asp Lys Asn Pro Met Pro Ala Pro Val Glu His Gln Ile Cys Pro
        435                 440                 445

Ala Val Cys Val Leu Met Lys Leu Ser Phe Asp Glu Glu His Arg His
    450                 455                 460

Ala Met Asn Glu Leu Gly Gly Leu Gln Ala Ile Ala Glu Leu Leu Gln
465                 470                 475                 480

Val Asp Cys Glu Met Tyr Gly Leu Thr Asn Asp His Tyr Ser Ile Thr
                485                 490                 495

Leu Arg Arg Tyr Ala Gly Met Ala Leu Thr Asn Leu Thr Phe Gly Asp
            500                 505                 510

Val Ala Asn Lys Ala Thr Leu Cys Ser Met Lys Gly Cys Met Arg Ala
        515                 520                 525

Leu Val Ala Gln Leu Lys Ser Glu Ser Glu Asp Leu Gln Gln Val Ile
    530                 535                 540

Ala Ser Val Leu Arg Asn Leu Ser Trp Arg Ala Asp Val Asn Ser Lys
545                 550                 555                 560

Lys Thr Leu Arg Glu Val Gly Ser Val Lys Ala Leu Met Glu Cys Ala
                565                 570                 575

Leu Glu Val Lys Lys Glu Ser Thr Leu Lys Ser Val Leu Ser Ala Leu
            580                 585                 590

Trp Asn Leu Ser Ala His Cys Thr Glu Asn Lys Ala Asp Ile Cys Ala
        595                 600                 605

Val Asp Gly Ala Leu Ala Phe Leu Val Gly Thr Leu Thr Tyr Arg Ser
    610                 615                 620

Gln Thr Asn Thr Leu Ala Ile Ile Glu Ser Gly Gly Gly Ile Leu Arg
625                 630                 635                 640

Asn Val Ser Ser Leu Ile Ala Thr Asn Glu Asp His Arg Gln Ile Leu
                645                 650                 655

Arg Glu Asn Asn Cys Leu Gln Thr Leu Leu Gln His Leu Lys Ser His
            660                 665                 670

Ser Leu Thr Ile Val Ser Asn Ala Cys Gly Thr Leu Trp Asn Leu Ser
        675                 680                 685

Ala Arg Asn Pro Lys Asp Gln Glu Ala Leu Trp Asp Met Gly Ala Val
    690                 695                 700

Ser Met Leu Lys Asn Leu Ile His Ser Lys His Lys Met Ile Ala Met
705                 710                 715                 720

Gly Ser Ala Ala Ala Leu Arg Asn Leu Met Ala Asn Arg Pro Ala Lys
                725                 730                 735

Tyr Lys Asp Ala Asn Ile Met Ser Pro Gly Ser Ser Leu Pro Ser Leu
            740                 745                 750

His Val Arg Lys Gln Lys Ala Leu Glu Ala Glu Leu Asp Ala Gln His
        755                 760                 765

Leu Ser Glu Thr Phe Asp Asn Ile Asp Asn Leu Ser Pro Lys Ala Ser
    770                 775                 780

His Arg Ser Lys Gln Arg His Lys Gln Ser Leu Tyr Gly Asp Tyr Val
785                 790                 795                 800

Phe Asp Thr Asn Arg His Asp Asp Asn Arg Ser Asp Asn Phe Asn Thr
                805                 810                 815

Gly Asn Met Thr Val Leu Ser Pro Tyr Leu Asn Thr Thr Val Leu Pro
            820                 825                 830

Ser Ser Ser Ser Ser Arg Gly Ser Leu Asp Ser Ser Arg Ser Glu Lys
        835                 840                 845

Asp Arg Ser Leu Glu Arg Glu Arg Gly Ile Gly Leu Gly Asn Tyr His
    850                 855                 860

Pro Ala Thr Glu Asn Pro Gly Thr Ser Ser Lys Arg Gly Leu Gln Ile
865                 870                 875                 880

Ser Thr Thr Ala Ala Gln Ile Ala Lys Val Met Glu Glu Val Ser Ala
                885                 890                 895

Ile His Thr Ser Gln Glu Asp Arg Ser Ser Gly Ser Thr Thr Glu Leu
            900                 905                 910

His Cys Val Thr Asp Glu Arg Asn Ala Leu Arg Arg Ser Ser Ala Ala
        915                 920                 925

His Thr His Ser Asn Thr Tyr Asn Phe Thr Lys Ser Glu Asn Ser Asn
    930                 935                 940

Arg Thr Cys Ser Met Pro Tyr Ala Lys Leu Glu Tyr Lys Arg Ser Ser
945                 950                 955                 960

Asn Asp Ser Leu Asn Ser Val Ser Ser Ser Asp Gly Tyr Gly Lys Arg
                965                 970                 975

Gly Gln Met Lys Pro Ser Ile Glu Ser Tyr Ser Glu Asp Asp Glu Ser
            980                 985                 990

Lys Phe Cys Ser Tyr Gly Gln Tyr Pro Ala Asp Leu Ala His Lys Ile
        995                 1000                1005

His Ser Ala Asn His Met Asp Asp Asn Asp Gly Glu Leu Asp Thr
    1010                1015                1020

Pro Ile Asn Tyr Ser Leu Lys Tyr Ser Asp Glu Gln Leu Asn Ser
    1025                1030                1035

Gly Arg Gln Ser Pro Ser Gln Asn Glu Arg Trp Ala Arg Pro Lys
    1040                1045                1050

His Ile Ile Glu Asp Glu Ile Lys Gln Ser Glu Gln Arg Gln Ser
    1055                1060                1065

Arg Asn Gln Ser Thr Thr Tyr Pro Val Tyr Thr Glu Ser Thr Asp
    1070                1075                1080

Asp Lys His Leu Lys Phe Gln Pro His Phe Gly Gln Gln Glu Cys
    1085                1090                1095

Val Ser Pro Tyr Arg Ser Arg Gly Ala Asn Gly Ser Glu Thr Asn
    1100                1105                1110

Arg Val Gly Ser Asn His Gly Ile Asn Gln Asn Val Ser Gln Ser
    1115                1120                1125

Leu Cys Gln Glu Asp Asp Tyr Glu Asp Asp Lys Pro Thr Asn Tyr
    1130                1135                1140

Ser Glu Arg Tyr Ser Glu Glu Glu Gln His Glu Glu Glu Glu Arg
    1145                1150                1155

Pro Thr Asn Tyr Ser Ile Lys Tyr Asn Glu Glu Lys Arg His Val
    1160                1165                1170

Asp Gln Pro Ile Asp Tyr Ser Leu Lys Tyr Ala Thr Asp Ile Pro
    1175                1180                1185

Ser Ser Gln Lys Gln Ser Phe Ser Phe Ser Lys Ser Ser Ser Gly
    1190                1195                1200

Gln Ser Ser Lys Thr Glu His Met Ser Ser Ser Ser Glu Asn Thr
    1205                1210                1215

Ser Thr Pro Ser Ser Asn Ala Lys Arg Gln Asn Gln Leu His Pro
    1220                1225                1230

Ser Ser Ala Gln Ser Arg Ser Gly Gln Pro Gln Lys Ala Ala Thr
    1235                1240                1245

Cys Lys Val Ser Ser Ile Asn Gln Glu Thr Ile Gln Thr Tyr Cys
    1250                1255                1260

Val Glu Asp Thr Pro Ile Cys Phe Ser Arg Cys Ser Ser Leu Ser
    1265                1270                1275

Ser Leu Ser Ser Ala Glu Asp Glu Ile Gly Cys Asn Gln Thr Thr
    1280                1285                1290

Gln Glu Ala Asp Ser Ala Asn Thr Leu Gln Ile Ala Glu Ile Lys
    1295                1300                1305

Glu Lys Ile Gly Thr Arg Ser Ala Glu Asp Pro Val Ser Glu Val
    1310                1315                1320

Pro Ala Val Ser Gln His Pro Arg Thr Lys Ser Ser Arg Leu Gln
    1325                1330                1335

Gly Ser Ser Leu Ser Ser Glu Ser Ala Arg His Lys Ala Val Glu
    1340                1345                1350

Phe Ser Ser Gly Ala Lys Ser Pro Ser Lys Ser Gly Ala Gln Thr
    1355                1360                1365

Pro Lys Ser Pro Pro Glu His Tyr Val Gln Glu Thr Pro Leu Met
    1370                1375                1380

Phe Ser Arg Cys Thr Ser Val Ser Ser Leu Asp Ser Phe Glu Ser
    1385                1390                1395

Arg Ser Ile Ala Ser Ser Val Gln Ser Glu Pro Cys Ser Gly Met
    1400                1405                1410

Val Ser Gly Ile Ile Ser Pro Ser Asp Leu Pro Asp Ser Pro Gly
    1415                1420                1425

Gln Thr Met Pro Pro Ser Arg Ser Lys Thr Pro Pro Pro Pro Pro
    1430                1435                1440

Gln Thr Ala Gln Thr Lys Arg Glu Val Pro Lys Asn Lys Ala Pro
    1445                1450                1455

Thr Ala Glu Lys Arg Glu Ser Gly Pro Lys Gln Ala Ala Val Asn
    1460                1465                1470

Ala Ala Val Gln Arg Val Gln Val Leu Pro Asp Ala Asp Thr Leu
    1475                1480                1485

Leu His Phe Ala Thr Glu Ser Thr Pro Asp Gly Phe Ser Cys Ser
    1490                1495                1500

Ser Ser Leu Ser Ala Leu Ser Leu Asp Glu Pro Phe Ile Gln Lys
    1505                1510                1515

Asp Val Glu Leu Arg Ile Met Pro Pro Val Gln Glu Asn Asp Asn
    1520                1525                1530

Gly Asn Glu Thr Glu Ser Glu Gln Pro Lys Glu Ser Asn Glu Asn
    1535                1540                1545

Gln Glu Lys Glu Ala Glu Lys Thr Ile Asp Ser Glu Lys Asp Leu
    1550                1555                1560

Leu Asp Asp Ser Asp Asp Asp Asp Ile Glu Ile Leu Glu Glu Cys
    1565                1570                1575

Ile Ile Ser Ala Met Pro Thr Lys Ser Ser Arg Lys Ala Lys Lys
    1580                1585                1590

Pro Ala Gln Thr Ala Ser Lys Leu Pro Pro Pro Val Ala Arg Lys
    1595                1600                1605

Pro Ser Gln Leu Pro Val Tyr Lys Leu Leu Pro Ser Gln Asn Arg
    1610                1615                1620

Leu Gln Pro Gln Lys His Val Ser Phe Thr Pro Gly Asp Asp Met
    1625                1630                1635

Pro Arg Val Tyr Cys Val Glu Gly Thr Pro Ile Asn Phe Ser Thr
    1640                1645                1650

Ala Thr Ser Leu Ser Asp Leu Thr Ile Glu Ser Pro Pro Asn Glu
    1655                1660                1665

Leu Ala Ala Gly Glu Gly Val Arg Gly Gly Ala Gln Ser Gly Glu
    1670                1675                1680

Phe Glu Lys Arg Asp Thr Ile Pro Thr Glu Gly Arg Ser Thr Asp
    1685                1690                1695

Glu Ala Gln Gly Gly Lys Thr Ser Ser Val Thr Ile Pro Glu Leu
    1700                1705                1710

Asp Asp Asn Lys Ala Glu Glu Gly Asp Ile Leu Ala Glu Cys Ile
    1715                1720                1725

Asn Ser Ala Met Pro Lys Gly Lys Ser His Lys Pro Phe Arg Val
    1730                1735                1740

Lys Lys Ile Met Asp Gln Val Gln Gln Ala Ser Ala Ser Ser Ser
    1745                1750                1755

Ala Pro Asn Lys Asn Gln Leu Asp Gly Lys Lys Lys Lys Pro Thr
    1760                1765                1770

Ser Pro Val Lys Pro Ile Pro Gln Asn Thr Glu Tyr Arg Thr Arg
    1775                1780                1785

Val Arg Lys Asn Ala Asp Ser Lys Asn Asn Leu Asn Ala Glu Arg
    1790                1795                1800

Val Phe Ser Asp Asn Lys Asp Ser Lys Lys Gln Asn Leu Lys Asn
    1805                1810                1815

Asn Ser Lys Asp Phe Asn Asp Lys Leu Pro Asn Asn Glu Asp Arg
    1820                1825                1830

Val Arg Gly Ser Phe Ala Phe Asp Ser Pro His His Tyr Thr Pro
    1835                1840                1845

Ile Glu Gly Thr Pro Tyr Cys Phe Ser Arg Asn Asp Ser Leu Ser
    1850                1855                1860

Ser Leu Asp Phe Asp Asp Asp Asp Val Asp Leu Ser Arg Glu Lys
    1865                1870                1875

Ala Glu Leu Arg Lys Ala Lys Glu Asn Lys Glu Ser Glu Ala Lys
    1880                1885                1890

Val Thr Ser His Thr Glu Leu Thr Ser Asn Gln Gln Ser Ala Asn
    1895                1900                1905

Lys Thr Gln Ala Ile Ala Lys Gln Pro Ile Asn Arg Gly Gln Pro
    1910                1915                1920

Lys Pro Ile Leu Gln Lys Gln Ser Thr Phe Pro Gln Ser Ser Lys
    1925                1930                1935

Asp Ile Pro Asp Arg Gly Ala Ala Thr Asp Glu Lys Leu Gln Asn
    1940                1945                1950

Phe Ala Ile Glu Asn Thr Pro Val Cys Phe Ser His Asn Ser Ser
    1955                1960                1965

Leu Ser Ser Leu Ser Asp Ile Asp Gln Glu Asn Asn Asn Lys Glu
    1970                1975                1980

Asn Glu Pro Ile Lys Glu Thr Glu Pro Pro Asp Ser Gln Gly Glu
    1985                1990                1995

Pro Ser Lys Pro Gln Ala Ser Gly Tyr Ala Pro Lys Ser Phe His
    2000                2005                2010

Val Glu Asp Thr Pro Val Cys Phe Ser Arg Asn Ser Ser Leu Ser
    2015                2020                2025

Ser Leu Ser Ile Asp Ser Glu Asp Asp Leu Leu Gln Glu Cys Ile
    2030                2035                2040

Ser Ser Ala Met Pro Lys Lys Lys Lys Pro Ser Arg Leu Lys Gly
    2045                2050                2055

Asp Asn Glu Lys His Ser Pro Arg Asn Met Gly Gly Ile Leu Gly
    2060                2065                2070

Glu Asp Leu Thr Leu Asp Leu Lys Asp Ile Gln Arg Pro Asp Ser
    2075                2080                2085

Glu His Gly Leu Ser Pro Asp Ser Glu Asn Phe Asp Trp Lys Ala
    2090                2095                2100

Ile Gln Glu Gly Ala Asn Ser Ile Val Ser Ser Leu His Gln Ala
    2105                2110                2115

Ala Ala Ala Ala Cys Leu Ser Arg Gln Ala Ser Ser Asp Ser Asp
    2120                2125                2130

Ser Ile Leu Ser Leu Lys Ser Gly Ile Ser Leu Gly Ser Pro Phe
    2135                2140                2145

His Leu Thr Pro Asp Gln Glu Glu Lys Pro Phe Thr Ser Asn Lys
    2150                2155                2160

Gly Pro Arg Ile Leu Lys Pro Gly Glu Lys Ser Thr Leu Glu Thr
    2165                2170                2175

Lys Lys Ile Glu Ser Glu Ser Lys Gly Ile Lys Gly Gly Lys Lys
    2180                2185                2190

Val Tyr Lys Ser Leu Ile Thr Gly Lys Val Arg Ser Asn Ser Glu
    2195                2200                2205

Ile Ser Gly Gln Met Lys Gln Pro Leu Gln Ala Asn Met Pro Ser
    2210                2215                2220

Ile Ser Arg Gly Arg Thr Met Ile His Ile Pro Gly Val Arg Asn
    2225                2230                2235

Ser Ser Ser Ser Thr Ser Pro Val Ser Lys Lys Gly Pro Pro Leu
    2240                2245                2250

Lys Thr Pro Ala Ser Lys Ser Pro Ser Glu Gly Gln Thr Ala Thr
    2255                2260                2265

Thr Ser Pro Arg Gly Ala Lys Pro Ser Val Lys Ser Glu Leu Ser
    2270                2275                2280

Pro Val Ala Arg Gln Thr Ser Gln Ile Gly Gly Ser Ser Lys Ala
    2285                2290                2295

Pro Ser Arg Ser Gly Ser Arg Asp Ser Thr Pro Ser Arg Pro Ala
    2300                2305                2310

Gln Gln Pro Leu Ser Arg Pro Ile Gln Ser Pro Gly Arg Asn Ser
    2315                2320                2325

Ile Ser Pro Gly Arg Asn Gly Ile Ser Pro Pro Asn Lys Leu Ser
    2330                2335                2340

Gln Leu Pro Arg Thr Ser Ser Pro Ser Thr Ala Ser Thr Lys Ser
    2345                2350                2355

Ser Gly Ser Gly Lys Met Ser Tyr Thr Ser Pro Gly Arg Gln Met
    2360                2365                2370

Ser Gln Gln Asn Leu Thr Lys Gln Thr Gly Leu Ser Lys Asn Ala
    2375                2380                2385

Ser Ser Ile Pro Arg Ser Glu Ser Ala Ser Lys Gly Leu Asn Gln
    2390                2395                2400

Met Asn Asn Gly Asn Gly Ala Asn Lys Lys Val Glu Leu Ser Arg
    2405                2410                2415

Met Ser Ser Thr Lys Ser Ser Gly Ser Glu Ser Asp Arg Ser Glu
    2420                2425                2430

Arg Pro Val Leu Val Arg Gln Ser Thr Phe Ile Lys Glu Ala Pro
    2435                2440                2445

Ser Pro Thr Leu Arg Arg Lys Leu Glu Glu Ser Ala Ser Phe Glu
    2450                2455                2460

Ser Leu Ser Pro Ser Ser Arg Pro Ala Ser Pro Thr Arg Ser Gln
    2465                2470                2475

Ala Gln Thr Pro Val Leu Ser Pro Ser Leu Pro Asp Met Ser Leu
    2480                2485                2490

Ser Thr His Ser Ser Val Gln Ala Gly Gly Trp Arg Lys Leu Pro
    2495                2500                2505

Pro Asn Leu Ser Pro Thr Ile Glu Tyr Asn Asp Gly Arg Pro Ala
    2510                2515                2520

Lys Arg His Asp Ile Ala Arg Ser His Ser Glu Ser Pro Ser Arg
    2525                2530                2535

Leu Pro Ile Asn Arg Ser Gly Thr Trp Lys Arg Glu His Ser Lys
    2540                2545                2550

His Ser Ser Ser Leu Pro Arg Val Ser Thr Trp Arg Arg Thr Gly
    2555                2560                2565

Ser Ser Ser Ser Ile Leu Ser Ala Ser Ser Glu Ser Ser Glu Lys
    2570                2575                2580

Ala Lys Ser Glu Asp Glu Lys His Val Asn Ser Ile Ser Gly Thr
    2585                2590                2595

Lys Gln Ser Lys Glu Asn Gln Val Ser Ala Lys Gly Thr Trp Arg
    2600                2605                2610

Lys Ile Lys Glu Asn Glu Phe Ser Pro Thr Asn Ser Thr Ser Gln
    2615                2620                2625

Thr Val Ser Ser Gly Ala Thr Asn Gly Ala Glu Ser Lys Thr Leu
    2630                2635                2640

Ile Tyr Gln Met Ala Pro Ala Val Ser Lys Thr Glu Asp Val Trp
    2645                2650                2655

Val Arg Ile Glu Asp Cys Pro Ile Asn Asn Pro Arg Ser Gly Arg
    2660                2665                2670

Ser Pro Thr Gly Asn Thr Pro Pro Val Ile Asp Ser Val Ser Glu
    2675                2680                2685

Lys Ala Asn Pro Asn Ile Lys Asp Ser Lys Asp Asn Gln Ala Lys
    2690                2695                2700

Gln Asn Val Gly Asn Gly Ser Val Pro Met Arg Thr Val Gly Leu
    2705                2710                2715

Glu Asn Arg Leu Asn Ser Phe Ile Gln Val Asp Ala Pro Asp Gln
    2720                2725                2730

Lys Gly Thr Glu Ile Lys Pro Gly Gln Asn Asn Pro Val Pro Val
    2735                2740                2745

Ser Glu Thr Asn Glu Ser Ser Ile Val Glu Arg Thr Pro Phe Ser
    2750                2755                2760

Ser Ser Ser Ser Ser Lys His Ser Ser Pro Ser Gly Thr Val Ala
    2765                2770                2775

Ala Arg Val Thr Pro Phe Asn Tyr Asn Pro Ser Pro Arg Lys Ser
    2780                2785                2790

Ser Ala Asp Ser Thr Ser Ala Arg Pro Ser Gln Ile Pro Thr Pro
    2795                2800                2805

Val Asn Asn Asn Thr Lys Lys Arg Asp Ser Lys Thr Asp Ser Thr
    2810                2815                2820

Glu Ser Ser Gly Thr Gln Ser Pro Lys Arg His Ser Gly Ser Tyr
    2825                2830                2835

Leu Val Thr Ser Val
    2840

<210> 2
<211> 912
<212> PRT
<213> APC Protein from amino-acids 959 to 1870

<400> 2

Ser Ser Asn Asp Ser Leu Asn Ser Val Ser Ser Ser Asp Gly Tyr Gly
1               5                   10                  15

Lys Arg Gly Gln Met Lys Pro Ser Ile Glu Ser Tyr Ser Glu Asp Asp
            20                  25                  30

Glu Ser Lys Phe Cys Ser Tyr Gly Gln Tyr Pro Ala Asp Leu Ala His
        35                  40                  45

Lys Ile His Ser Ala Asn His Met Asp Asp Asn Asp Gly Glu Leu Asp
    50                  55                  60

Thr Pro Ile Asn Tyr Ser Leu Lys Tyr Ser Asp Glu Gln Leu Asn Ser
65                  70                  75                  80

Gly Arg Gln Ser Pro Ser Gln Asn Glu Arg Trp Ala Arg Pro Lys His
                85                  90                  95

Ile Ile Glu Asp Glu Ile Lys Gln Ser Glu Gln Arg Gln Ser Arg Asn
            100                 105                 110

Gln Ser Thr Thr Tyr Pro Val Tyr Thr Glu Ser Thr Asp Asp Lys His
        115                 120                 125

Leu Lys Phe Gln Pro His Phe Gly Gln Gln Glu Cys Val Ser Pro Tyr
    130                 135                 140

Arg Ser Arg Gly Ala Asn Gly Ser Glu Thr Asn Arg Val Gly Ser Asn
145                 150                 155                 160

His Gly Ile Asn Gln Asn Val Ser Gln Ser Leu Cys Gln Glu Asp Asp
                165                 170                 175

Tyr Glu Asp Asp Lys Pro Thr Asn Tyr Ser Glu Arg Tyr Ser Glu Glu
            180                 185                 190

Glu Gln His Glu Glu Glu Glu Arg Pro Thr Asn Tyr Ser Ile Lys Tyr
        195                 200                 205

Asn Glu Glu Lys Arg His Val Asp Gln Pro Ile Asp Tyr Ser Leu Lys
    210                 215                 220

Tyr Ala Thr Asp Ile Pro Ser Ser Gln Lys Gln Ser Phe Ser Phe Ser
225                 230                 235                 240

Lys Ser Ser Ser Gly Gln Ser Ser Lys Thr Glu His Met Ser Ser Ser
                245                 250                 255

Ser Glu Asn Thr Ser Thr Pro Ser Ser Asn Ala Lys Arg Gln Asn Gln
            260                 265                 270

Leu His Pro Ser Ser Ala Gln Ser Arg Ser Gly Gln Pro Gln Lys Ala
        275                 280                 285

Ala Thr Cys Lys Val Ser Ser Ile Asn Gln Glu Thr Ile Gln Thr Tyr
    290                 295                 300

Cys Val Glu Asp Thr Pro Ile Cys Phe Ser Arg Cys Ser Ser Leu Ser
305                 310                 315                 320

Ser Leu Ser Ser Ala Glu Asp Glu Ile Gly Cys Asn Gln Thr Thr Gln
                325                 330                 335

Glu Ala Asp Ser Ala Asn Thr Leu Gln Ile Ala Glu Ile Lys Glu Lys
            340                 345                 350

Ile Gly Thr Arg Ser Ala Glu Asp Pro Val Ser Glu Val Pro Ala Val
        355                 360                 365

Ser Gln His Pro Arg Thr Lys Ser Ser Arg Leu Gln Gly Ser Ser Leu
    370                 375                 380

Ser Ser Glu Ser Ala Arg His Lys Ala Val Glu Phe Ser Ser Gly Ala
385                 390                 395                 400

Lys Ser Pro Ser Lys Ser Gly Ala Gln Thr Pro Lys Ser Pro Pro Glu
                405                 410                 415

His Tyr Val Gln Glu Thr Pro Leu Met Phe Ser Arg Cys Thr Ser Val
            420                 425                 430

Ser Ser Leu Asp Ser Phe Glu Ser Arg Ser Ile Ala Ser Ser Val Gln
        435                 440                 445

Ser Glu Pro Cys Ser Gly Met Val Ser Gly Ile Ile Ser Pro Ser Asp
    450                 455                 460

Leu Pro Asp Ser Pro Gly Gln Thr Met Pro Pro Ser Arg Ser Lys Thr
465                 470                 475                 480

Pro Pro Pro Pro Pro Gln Thr Ala Gln Thr Lys Arg Glu Val Pro Lys
                485                 490                 495

Asn Lys Ala Pro Thr Ala Glu Lys Arg Glu Ser Gly Pro Lys Gln Ala
            500                 505                 510

Ala Val Asn Ala Ala Val Gln Arg Val Gln Val Leu Pro Asp Ala Asp
        515                 520                 525

Thr Leu Leu His Phe Ala Thr Glu Ser Thr Pro Asp Gly Phe Ser Cys
    530                 535                 540

Ser Ser Ser Leu Ser Ala Leu Ser Leu Asp Glu Pro Phe Ile Gln Lys
545                 550                 555                 560

Asp Val Glu Leu Arg Ile Met Pro Pro Val Gln Glu Asn Asp Asn Gly
                565                 570                 575

Asn Glu Thr Glu Ser Glu Gln Pro Lys Glu Ser Asn Glu Asn Gln Glu
            580                 585                 590

Lys Glu Ala Glu Lys Thr Ile Asp Ser Glu Lys Asp Leu Leu Asp Asp
        595                 600                 605

Ser Asp Asp Asp Asp Ile Glu Ile Leu Glu Glu Cys Ile Ile Ser Ala
    610                 615                 620

Met Pro Thr Lys Ser Ser Arg Lys Ala Lys Lys Pro Ala Gln Thr Ala
625                 630                 635                 640

Ser Lys Leu Pro Pro Pro Val Ala Arg Lys Pro Ser Gln Leu Pro Val
                645                 650                 655

Tyr Lys Leu Leu Pro Ser Gln Asn Arg Leu Gln Pro Gln Lys His Val
            660                 665                 670

Ser Phe Thr Pro Gly Asp Asp Met Pro Arg Val Tyr Cys Val Glu Gly
        675                 680                 685

Thr Pro Ile Asn Phe Ser Thr Ala Thr Ser Leu Ser Asp Leu Thr Ile
    690                 695                 700

Glu Ser Pro Pro Asn Glu Leu Ala Ala Gly Glu Gly Val Arg Gly Gly
705                 710                 715                 720

Ala Gln Ser Gly Glu Phe Glu Lys Arg Asp Thr Ile Pro Thr Glu Gly
                725                 730                 735

## Claims

1. A transgenic *Drosophila* whose genome comprises the full-length human colon cancer gene *Adenomatous Polyposis Coli* (APC) having SEQ ID NO. 1 wherein:
(a) said genomic alteration allows mis-expression of full-length human APC in flies in regulated manner,
(b) said mis-expression of the full-length human APC results in developmental abnormalities,
(c) said developmental abnormalities induced by the mis-expression of full-length human APC in flies are similar to those exhibited by flies carrying mutations in *Drosophila wingless* gene, and
(d) to use the same as an assay system for screening and validating efficacy of drugs.

2. The transgenic *Drosophila* as claimed in claim 1 wherein, its genome includes β-catenin binding domain comprising of amino-acids from 959 to 1870 of SEQ ID NO. 2 from the full length human APC gene of SEQ ID NO.1, and this engineered disruption of human APC comprises only the five of the seven β-catenin binding domains wherein:
(a) said genomic alteration allows mis-expression of a truncated version of human APC in flies in a regulated manner,
(b) said mis-expression of the said gene construct results in the developmental abnormalities,
(c) said developmental abnormalities induced by the mis-expression of the said gene construct in dies is similar to those exhibited by flies carrying mutations in *Drosophila wingless* gene.
(d) said mis-expression of the said novel construct in regulated manner results in a more severe developmental phenotype, and
(e) to use the same as an assay system for screening and validating efficacy of drugs.

3. The transgenic *Drosophila* as claimed in claim I wherein, the N terminal domain of APC with amino acids from I to 767 having SEQ ID NO. 3, from the full length human APC gene of SEQ ID NO.1, wherein:
(a) said genomic alteration allows mis-expression of human APC in dies in a regulated manner,
(b) said mis-expression of the said novel construct in a regulated manner resulting in severe abnormalities in fly development during metamorphosis, and
(c) to use the same as an assay system for screening and validating efficacy of drugs.

4. A method for selecting a compound for pharmacological activity, which potentially inhibits or enhances the developmental abnormalities induced by the expression of full length and protein domains of human APC in *Drosophila*, said method comprising:
(a) providing the first, second, and third transgenic fly of claims 1, 2 and 3 respectively, wherein said flies have said developmental abnormalities,
(b) administering the said compounds to the said transgenic *Drosophila* at different concentrations, and
(c) screening for the changes in the severity of the phenotype .

5. A method of determining various *Drosophila* proteins interacting with full-length and protein domains human APC protein wherein, said method comprising:
(a) providing the first, second, and third transgenic fly of claims 1, 2 and 3 respectively, wherein said flies have said developmental abnormalities,
(b) crossing the said transgenic flies individually to a set of *Drosophila* strains each of which carries mutation in a different gene or set of genes, and
(c) Screening for the change in the severity of the phenotype.

6. A method for determining the modulation and differential expression of genes following the mis-expression of full-length and its protein domains human APC in *Drosophila* wherein, said method comprising:
(a) providing the transgenic *Drosophila* as claimed in claims 1,2 and 3 wherein, the flies have developmental abnormalities,
(b) screening for differential gene expression using differential display-RT PCR or microarray techniques, and
(c) identifying genes that are differentially regulated on expression of human APC.

7. A method for determining the modulation and differential expression of proteins following the mis-expression of full-length and its protein domain human APC in *Drosophila* wherein, said method comprising:
(a) providing the transgenic *Drosophila* , as claimed in claims 1, 2 and 3 wherein, the flies have developmental abnormalities,
(b) identifying differential gene expression and protein modifications using proteomics techniques, and
(c) identifying gene products that are differentially regulated on expression of human APC.

8. A method to study Wnt/Wg signaling in *Drosophila* said method comprising;
(a) providing the transgenic *Drosophila*, as claimed in claims 1, 2 and 3,
(b) crossing these transgenic flies to a number of GAL4 drivers to induce targeted expression of said constructs in various tissues and at different developmental stages, and
(c) examining developmental abnormalities.

9. Methods as claimed in claims 6-8 wherein, examination of developmental abnormalities using gain-of-function genetic model for human APC to study mechanism of various developmental processes such as wing, leg, eye, antennae, and adult cuticle development.

10. A Method as claimed in claim 4 wherein, screening and validating efficacy of preventive and therapeutic drugs following APC gene mis-expression.

11. A Method as claimed in claim 4 wherein, human APC pathway is identified using drug selected from a group of compunds comprising anti inflammatory, Analgesics, Antipyretics, and Antineoplastics.

12. A method as claimed in claim 4 wherein, concentration of said drugs ranging between 50 to 500 µg/ml of fly food.

13. Methods as claimed claims 6-8 wherein, examination of developmental abnormalities using gain-of-function genetic model for human APC which has advantages to study the *Drosophila* Wnt/Wg signaling pathway.

14. A Method as claimed in claim 8 wherein, studying the kinetics of Wnt/Wg signaling during various developmental stages and in different tissues.

15. A Method as claimed in claims 5 and 7 wherein, new target proteins interacting with β-catenin are identified.

16. A Method as claimed in claim 6 wherein, genes interacting with APC are identified.

17. Methods as claimed in claims 5-8 wherein, examination of developmental abnormalities using gain-of-function genetic model for human APC to study biochemical function of human APC function.

18. Methods as claimed in claims 5-8 wherein, examination of developmental abnormalities using gain-of-function genetic model for human APC to identify additional components of *Drosophila* Wnt/Wg signaling pathway.

19. A transgenic *Drosophila* whose genome comprises the full-length human colon cancer gene *Adenomatous Polyposis Coli* (APC) having SEQ ID NO.1 wherein:
(a) said genomic alteration allows mis-expression of full-length human APC in flies in regulated manner,
(b) said mis-expression of the full-length human APC results in developmental abnormalities,
(c) said developmental abnormalities induced by the mis-expression of full-length human APC in flies are similar to those exhibited by flies carrying mutations in *Drosophila wingless* gene, and
(d) to use the same as an assay system for screening and validating efficacy of anti-cancer drugs.

20. The transgenic *Drosophila* as claimed in claim 19 wherein, its genome includes β-catenin binding domain comprising of amino-acids from 959 to 1870 of SEQ ID NO. 2 from the full length human APC gene of SEQ ID NO.1, and this engineered disruption of human APC comprises only the five of the seven β-catenin binding domains wherein:
(a) said genomic alteration allows mis-expression of a truncated version of human APC in flies in a regulated manner,
(b) the mis-expression of the said gene construct results in the developmental abnormalities,
(c) the developmental abnormalities induced by the mis-expression of the said gene construct in flies is similar to those exhibited by flies carrying mutations in *Drosophila wingless* gene,
(d) mis-expression of the said novel construct in regulated manner results in a more severe developmental phenotype, and
(e) to use the same as an assay system for screening and validating efficacy of anti-cancer drugs.

21. The transgenic *Drosophila* as claimed in claim 19 wherein, the N terminal domain of APC with amino acids from 1 to 767 having SEQ ID NO. 3, from the full length human APC gene of SEQ ID NO.1 wherein:
(a) the said genomic alteration allows mis-expression of human APC in flies in a regulated manner,
(b) the mis-expression of the said novel construct in a regulated manner resulting in severe abnormalities in fly development during metamorphosis, and
(c) to use the same as an assay system for screening and validating efficacy of anti-cancer drugs.

22. A method for selecting a compound for anti-cancer activity, which potentially inhibits or enhances the developmental abnormalities induced by the expression of full length and protein domains of human APC in *Drosophila*, said method comprising:
(a) providing the first, second, and third transgenic fly of claims 19, 20, and 21 respectively, wherein said flies have said developmental abnormalities,
(b) administering the said compounds to the said transgenic *Drosophila* at different concentrations, and
(c) screening for the change in the severity of the phenotype .

23. A method of determining various *Drosophila* proteins interacting with full-length and protein domains human APC protein wherein, said method comprising:
(a) providing the first, second, and third transgenic fly of claims 19, 20, and 21 respectively, wherein said flies have said developmental abnormalities,
(b) crossing the said transgenic flies individually to a set of *Drosophila* strains each of which carries mutation in a different gene or set of genes, and
(c) Screening for the change in the severity of the phenotype.

24. A method for determining the modulation and differential expression of genes following the mis-expression of full-length and its protein domains human APC in *Drosophila* wherein, said method comprising:
(a) providing the transgenic *Drosophila* as claimed in claims 19,20, and 21 wherein, the flies have developmental abnormalities,
(b) screening for differential gene expression using differential display-RT PCR or microarray techniques, and
(c) identifying genes that are differentially regulated on expression of human APC.

25. A method for determining the modulation and differential expression of proteins following the mis-expression of full-length and its protein domain human APC in *Drosophila* wherein, said method comprising:
(a) providing the transgenic *Drosophila*, as claimed in claims 19, 20, and 21 wherein, the flies have developmental abnormalities,
(b) identifying differential gene expression and protein modifications using proteomics techniques, and
(c) identifying gene products that are differentially regulated on expression of human APC.

26. A method to study Wnt/Wg signaling in *Drosophila* said method comprising;
(a) providing the transgenic *Drosophila*, as claimed in claims 19-21,
(b) crossing these transgenic flies to a number of GAL4 drivers to induce targeted expression of said constructs in various tissues and at different developmental stages, and
(c) examining developmental abnormalities.

27. Methods as claimed in claims 24-26 wherein, examination of developmental abnormalities using gain-of-function genetic model for human APC to study mechanism of various developmental processes such wing, leg, eye, antennae, and adult cuticle development.

28. A Method as claimed in claim 22 wherein, screening and validating efficacy of anti-cancer drugs following APC gene mis-expression.

29. A Method as claimed in claim 22 wherein, human APC pathway is identified using drugs selected from a group of compounds comprising anti inflammatory, Analgesics, Antipyretics, and Antineoplastics.

30. A method as claimed in claim 22 wherein, concentration of said anti-cancer drugs ranging between 50 to 500 µg/ml of fly food.

31. Methods as claimed claims 24-26 wherein, examination of developmental abnormalities using gain-of-function genetic model for human APC which has advantages to study the *Drosophila* Wnt/Wg signaling pathway.

32. A Method as claimed in claim 26 wherein, studying the kinetics of Wnt/Wg signaling during various developmental stages and in different tissues.

33. Methods as claimed in claims 23 and 25 wherein, new target proteins interacting with β-catenin are identified.

34. A Method as claimed in claim 24 wherein, genes interacting with APC are identified.

35. Methods as claimed in claims 23-26 wherein, examination of developmental abnormalities using gain-of-function genetic model for human APC to study biochemical function of human APC function.

36. Methods as claimed in claims 23-26 wherein, examination of developmental abnormalities using gain-of-function genetic model for human APC to identify additional components of *Drosophila* Wnt/Wg signaling pathway.
